# EUROPEAN PATENT SPECIFICATION

(11) **EP 1 183 040 B1**
(45) Date of publication and mention of the grant of the patent: **01.12.2004**
(21) Application number: 00935413.5
(22) Date of filing: 05.06.2000
(51) Int. Cl.: A61K 38/17, A61K 39/395, A61K 48/00, G01N 33/53, C12N 15/63, A01K 67/027, A61P 11/00, A61P 13/12, A61P 35/00, A61P 37/00

(54) **THERAPEUTIC USE OF AN INHIBITOR OF A HEDGEHOG SIGNALLING PATHWAY**
THERAPEUTISCHE VERWENDUNG EINES INHIBITORS DES HEDGEHOG SIGNALÜBERTRAGUNGSWEGES
UTILISATION THERAPEUTIQUE D'UN INHIBITEUR DU SIGNAL HEDGEHOG

(30) Priority: 08.06.1999 GB 9913350; 16.09.1999 GB 9921953
(43) Date of publication of application: 06.03.2002
(73) Proprietor: Lorantis Limited, Cambridge CB4 0PE (GB)
(72) Inventor: Lamb, Jonathan Robert, Edinburgh EH8 9AG (GB); Hoyne, Gerard Francis, Edinburgh EH8 9AG (GB); Dallman, Margaret Jane, London SW7 2AZ (GB)
(74) Representative: Mallalieu, Catherine Louise
(86) International application number: PCT/GB2000/002191
(87) International publication number: WO 2000/074706

(56) References cited:
- EP-A- 0 874 048
- WO-A-99/22000
- WO-A-99/52534
- WO-A-99/63052
- J. DUMONTELLE ET AL.: "Inhibition of expression of the endogenous mammary oncogene Wnt-1 by antisense oligodeoxynucleotides." PROCEEDINGS OF THE AMERICAN ASSOCIATION FOR CANCER RESEARCH, vol. 36, March 1995 (1995-03), page 515 XP002147584 USA
- E. FUJITA ET AL.: "Involvement of sonic hedgehog in the cell growth of LK-2 cells, human lung squamous carcinoma cells." BIOCHEMICAL AND BIOPHYSICAL RESEARCH COMMUNICATIONS, vol. 238, no. 2, 18 September 1997 (1997-09-18), pages 658-664, XP002147585 Duluth, MN, USA
- M. MURONE ET AL.: "Sonic hedgehog signaling by the patched-smoothened receptor complex." CURRENT BIOLOGY, vol. 9, no. 2, 28 January 1999 (1999-01-28), pages 76-84, XP000929784
- J. POLANEC ET AL.: "Effect of Wnt-1 antisense RNA on the outgrowth of a mammary adenocarcinoma cell line expressing that oncogene." CLINICAL MOLECULAR PATHOLOGY, vol. 49, no. 3, 1996, pages M166-M169, XP000929749

## Description

### Field of the Invention

The present relates to the use of an inhibitor of a Hedgehog signalling pathway in the preparation of a medicament for treatment of epithelial cell hyperplasia, fibrosis of tissue, inflammation or an immune disorder.

### Background

In many tissues, such as the lung and kidney, chronic unresolving inflammation may lead to remodelling in which both epithethial cell hyperplasia and fibrosis occur. In addition, there is an accompanying mononuclear cell infiltration at local sites of inflammation and the induction of immune responses reactive with self antigens. Similar pathology is also observed in chronic rejection of transplanted organs. In general, these diseases are difficult to manage clinically and this is well illustrated by chronic obstructive pulmonary disease where current pharmacological intervention has limited effects. Therefore, the ability to control this dysregulation of epithelial repair processes that drive anti-self immune responses and tissue remodelling will have an important clinical impact.

### Summary of the Invention

The formation of epithelial surfaces and the regulation of epithelial cell growth appears to be highly conserved among species. Recent studies in developmental biology have demonstrated the importance of epithelial cell growth factor genes Shh and Wnt 1 and the TGF-β superfamily members BMP4 and BMP7. These gene products not only play an important role in developmental epithelial cell growth but also in tissue patterning. The latter requires the coordinated and temporal regulation of cell differentiation programmes. During development cells respond to growth factors which are found within their environment and it is the interpretation of these various growth factor signalling pathways that will determine the differentiation programme that is initiated. To date little is known of the contribution of these genes in tissue remodelling and fibrosis that are observed in chronic inflammation; although BMP has been implicated in fibrosis in bone disease. We have noted that in diseased lungs there is increased expression of the gene *patched* which is involved in Hedgehog signalling. We have also noted that intratracheal instillation of plasmid DNA for the Hedgehog gene leads to the development of epithelial cell hyperplasia, fibrosis of tissue and the infiltration of mononuclear cells. The pathology is similar to that observed in interstitial lung disease.

We propose that antagonists of components of Hedgehog signalling and/or antagonists of components of Hedgehog signalling may prevent and/or reverse diseases such as epithelial cell hyperplasia, tissue fibrosis, chronic inflammation and also prevent graft rejection.

### Statements of Invention

In one aspect the present invention provides the use of an inhibitor of a Hedgehog signalling pathway for the preparation of a medicament for the treatment epithelial cell hyperplasia, fibrosis of tissue, inflammation, or an immune disorder.

Put another way the present invention provides use of an antagonist of a component of a Hedgehog family member signalling pathway in the preparation of a medicament for the treatment of epithelial cell hyperplasia, fibrosis of tissue, inflammation, or an immune disorder.

In one embodiment the Hedgehog family member is Sonic hedgehog, Indian hedgehog or Desert hedgehog.

In one embodiment the pathway which is a target of Hedgehog signalling is a BMP signalling pathway or a Wnt signalling pathway.

In one embodiment the antagonist is HIP, cyclopamine, Fzb, Cerberus, WIF-1, Xnr-3 Noggin, Chordin, Gremlin, or Follistatin or a derivative, fragment, variant, mimetic, homologue or analogue thereof.

In another embodiment the antagonist is an antibody to a component of the Hedgehog signalling pathway.

In a further embodiment the antagonist is itself a component of the Hedgehog signalling pathway.

Preferably the use of the present invention relates to the treatment of pulmonary hyperplasia or pulmonary fibrosis.

More preferably the use of the present invention relates to the treatment of adult respiratory distress syndrome; chronic obstructive airway disorders including asthma, emphysema and chronic bronchitis; atelectasis; occupational lung disease including silicosis; hypersensitivity diseases of the lung including hypersensitivity pneomonitis; idiopathic interstitial lung diseases including idiopathic pulmonary fibrosis, pneumonia including usual interstitial pneumonia, desquamative interstitial pneumonia and acute interstitial pneumonia; and pleural fibrosis.

In one embodiment the immune disorder is an autoimmune disease or graft rejection.

More particularly, the autoimmune disease may be thyroiditis, insultitis, multiple sclerosis, iridocyclitis, uveitis, orchitis, hepatitis, Addison's disease, myasthenia gravis, rheumatoid arthritis and lupus erythematosus.

In another aspect the present invention provides, a composition for use in treatment of epithelial cell hyperplasia, fibrosis of tissue, inflammation, cancer or an immune disorder comprising a therapeutically effective amount of an antagonist of a Hedgehog signalling pathway or an antagonist of the target pathway of Hedgehog signalling and a pharmaceutically acceptable carrier, diluent or excipient.

Various preferred features and embodiments of the present invention will now be described by way of non-limiting example and with reference to the accompanying drawings in which:
- Figure 1: shows a schematic representation of HH signalling;
- Figure 2: shows a schematic representation of a component of HH signalling;
- Figure 3: shows a schematic representation of Wnt signalling;
- Figure 4: shows plasmid SPC-SV40;
- Figure 5: shows plasmid SPC-Shh;

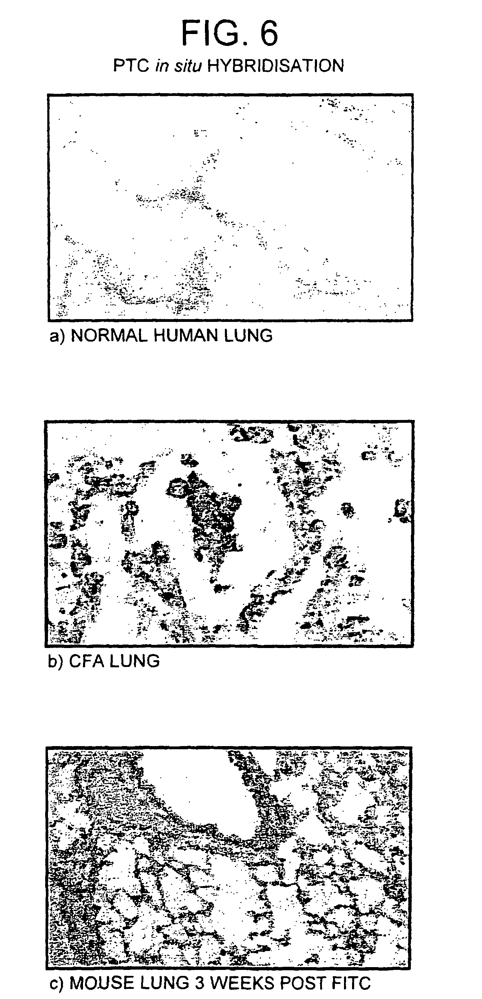

- Figure 6: show the results of Example 2;
- Figures 7-9: show the results of Example 3;
- Figure 10: show the results of Example 4;
- Figure 11: show the results of Example 5;
- Figure 12: show the results of Example 6;
- Figure 13: show the results of Example 7;
- Figure 14: show the results of Example 8;
- Figure 15: show the results of Example 9;
- Figure 16: show the results of Example 12; and
- Figure 17: show the results of Example 13.

For ease of reference a summary of the accompanying sequence listings is given below:
SEQ ID NO:1 shows the deduced amino acid sequence of mouse SHH and SEQ ID NO:2 shows the corresponding nucleic acid sequence;
SEQ ID NO:3 shows the deduced amino acid sequence of mouse Dvl-1 and
SEQ ID NO:4 shows the corresponding nucleic acid sequence;
SEQ ID NO:5 shows the deduced amino acid sequence of mouse HIP and SEQ ID NO:6 shows the corresponding nucleic acid sequence; and
SEQ ID NO:7 shows the deduced amino acid sequence of mouse WIF-1 and
SEQ ID NO:8 shows the corresponding nucleic acid sequence.

### Hedgehog Family Proteins

All multicellular organisms require cell communication to regulate growth and differentiation in the embryo. One strategy for this is to establish discrete organising centres that emit signals to coordinately control cell proliferation and cell fate determination. The *hedgehog (hh)* gene was identified originally through the segment polarity phenotype caused by its mutation in *Drosophila.* Genes of the *hh* family have now been isolated from several vertebrate species, including mouse, chicken, zebrafish, rat, *Xenopus* and human. The genes not only seem to show a high degree of structural homology both within and between species, but in addition exhibit some remarkable similarities in the ways in which they function in various embryonic processes. In vertebrates, *Sonic hedgehog* (*Shh*) is a key signal in several signalling centres. There are two other mammalian HH members, *Indian hedgehog (Ihh)* and *Desert hedgehog (Dhh)*.

A summary of various *hedgehog* genes is given in the following Table 1:

**Table 1**

| **Gene** | **Species** |
|---|---|
| *hedgehog (hh)* | *Drosophila* |
| *Sonic hedgehog (Shh)* | Mouse, Human, Rat, *Xenopus,* Chicken, Zebrafish |
| *Indian hedgehog (Ihh)* | Mouse, Human, Chicken |
| *Desert hedgehog (Dhh)* | Mouse |
| *Banded hedgehog (X-bhh)* | *Xenopus* |
| *Cepalic hedgehog (X-chh)* | *Xenopus* |
| *tiggy-winkle hedgehog (twhh)* | Zebrafish |
| *echidna hedgehog (ehh)* | Zebrafish |

The classification of genes from different species is based on the comparison of the expression pattern and the amino acid sequence. Of all vertebrate proteins, DHH is most similar to *Drosophila* HH (51% identity over entire length of processed proteins). Amino acid identity among SHH is 93% between mouse and human, 84% between mouse and chicken, 78% between mouse and *Xenopus*, and 68% between mouse and zebrafish. Intraspecies comparison within the mouse reveals 58-63% identity in pairwise combination between SHH, IHH and DHH. Interspecies comparison between the mouse and *Xenopus* reveals highest identities between IHH and XBHH (70%) and DHH and XCHH (64%).

The various Hedgehog proteins consist of a signal peptide, with a highly conserved N-terminal region and a more divergent C-terminal domain. It is understood that the biologically active Hedgehog peptides are formed from a larger precursor protein. In addition to signal sequence cleavage in the secretory pathway, Hedgehog precursor proteins undergo an internal autoproteolytic cleavage. This autocleavage generates an N-terminal peptide ( about 19kDa) and a C-terminal peptide (of about 26-28kDa). It is this N-terminal peptide that is necessary for short- and long-range Hedgehog signalling activities in *Drosophila* and vertebrates. The N-terminal peptide stays tightly associated with the surface of cells in which it is synthesised, while the C-terminal peptide is freely diffusable.

### Signalling Pathway

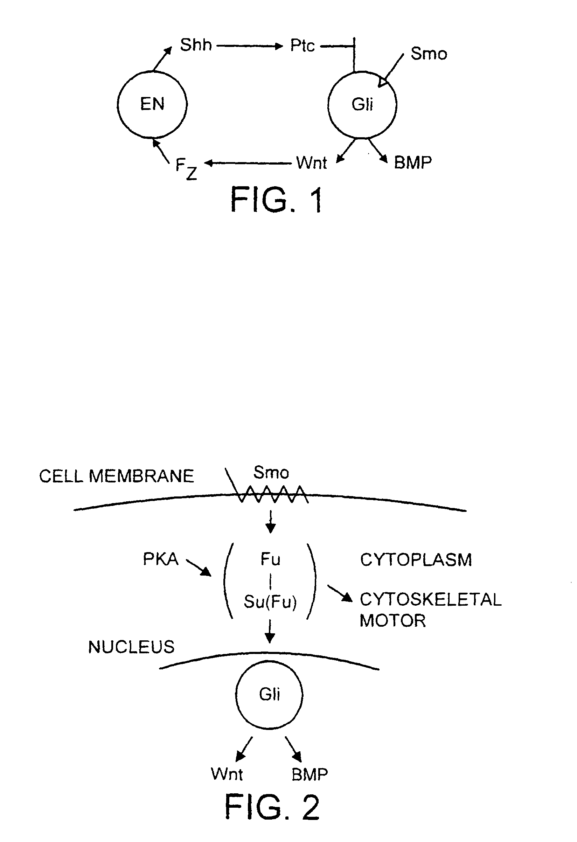

Figure 1 shows one representation of a Hedgehog signalling pathway, with particular reference to signalling in vertebrates.

Epithelial cells may express the homeodomain transcription factor engrailed (*en*) and secrete Hedgehog protein shown for illustrative purposes in the Figure as Shh. We have observed that En plays an important role in the maintainance of lymphocyte survival in the peripheral immune system.

In target cells, HH signalling is mediated by two transmembrane proteins patched (Ptc) which has structural similarities to channel and transporter proteins, and Smoothened (Smo), a seven-transmembrane protein similar to G-protein coupled receptors and the Wingless receptor Frizzeled (described below). Smo is a constitutive activator of HH target genes. Its activity is normally repressed by Ptc, and this repression is relieved by HH binding to Ptc. Thus, binding of HH to Ptc allows signal transduction leading to activation of the transcription factor Gli, which is located in the nucleus of the target cells.

The signal reaches Gli through the cytoplasmic complex formed between (1) the serine/threonine kinase Fused (Fu), (2) Suppressor of Fused (SU(Fu)); and (3) Costal2 (Cos2). Signalling through this complex may be inhibited by the cAMP-dependent protein kinase A (PKA) (see Figure 2).

Gli acts on target genes wingless (Wnt) and the BMP /activin growth factors. Both Wnt and BMP are secreted to the extracellular fluid to bind to their receptors. This process is illustrated schematically in Figure 1.

A summary and comparison of components of the Hedgehog signalling pathway is given below in Table 2:

**Table 2**

| *Drosophila* | Vertebrate |
|---|---|
| En | En 1,2 |
| hh | Ihh, Dhh, Shh |
| Ptc | Ptc 1,2 |
| Smo | Smo |
| Ci | Gli 1-3 |
| Target genes | |
| Wg | Wnt ∼15 |
| Dpp ≡ TGF_{β} | BMP 8-10 |

The nomenclature may be used interchangeably herein.

Further information on Hedgehog signalling may be find in the following articles: Ingham; Chuang and McMahon; Pepicelli *et al*; and Hammerschmidt *et al.*

### BMP signalling pathway

Bone morphogenic proteins (BMPs) are multifunctional cytokines, which are members of the transforming growth factor-β (TGF-β) superfamily. They regulate cellular proliferation, differentiation, apoptosis of various cells types. Activities of BMPs are extracellularly regulated by BMP-binding proteins, Noggin, Chordin, Gremlin Cerberus and Xnr-3. BMPs have been found to block neurogenesis in early development, but this can be resolved by soluble factors, e.g. Noggin and Chordin, which are secreted extracellularly and which bind and neutralize the BMPs preventing them from activating their receptors. In addition, signalling through BMP receptors can inhibit expression of the Notch ligand Delta, which we have previously shown to be important in regulating peripheral immune responses. The Notch signalling pathway is discussed in our International patent publication No. WO98/20142.

BMPs bind to two different types of serine-threonine kinase receptors, type I and type II. As for type I receptors, BMPs bind to BMP type IA receptor, BMP type IB receptor and activin type I receptor. As for type II receptors, BMPs bind to BMP type II receptor, activin type II receptor and activin type IIB receptor. In the receptor-ligand complexes, type II receptors phosphorylate type I receptors in the GS domain (rich in glycine, serine and threonine residues) to activate the latter. The activated type I receptors phosphorylate Smad family, which transduces the signals from cytoplasm into nuclei. Smad1, Smad5 and possibly MADH6 are activated by BMP receptors, form heteromeric complexes with Smad4, and translocate into the nucleus where they may activate transcription of various genes. Smad6 and Smad7 are inhibitory Smads, and may act as autocrine switch-off signals. BMP induced Smad signalling down regulates achaete/scute gene expression which is required for expression of the Notch ligand Delta. As indicated in Table 2 above, in *Drosophila,* Decapentaplegic (Dpp) is a homolog of mammalian BMPs.

### Wingless/Wnt signalling pathway

We have examined the role for dysregulation of the Wnt signalling pathway in interstitial lung disease. The Wnt genes are targets of the HH pathway, and the Wnt proteins are secreted growth factors which are involved in the regulation of epithelial cell proliferation and differentiation in the lung during embryonic development. We propose that Wnt signalling may also be upregulated during processes of epithelial cell repair in the lung.

Dishevelled-1 (Dvl-1) is the murine homolog of the fly Dsh gene and functions to transmit signals from the Wnt receptor, Frizzled, to the cytoplasm, where it regulates the kinase activity of a well known serine/threonine kinase, GSK-3b. Over expression of Dsh in fly epithelia leads to oncogenic activation of the epithelium by increasing Wnt signalling.

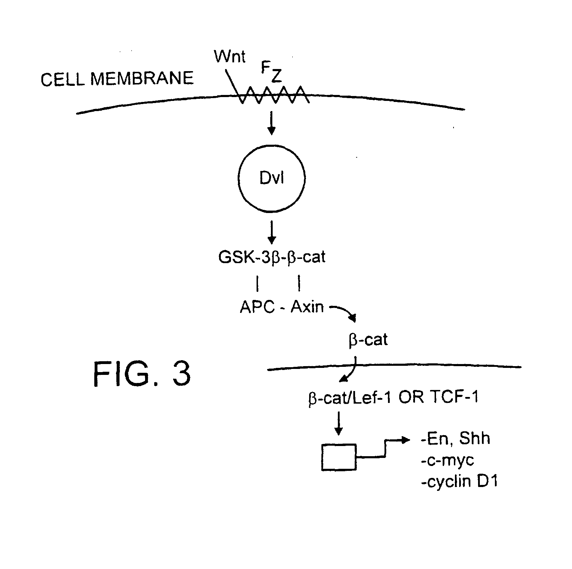

A representation of this pathway is shown in Figure 3. Wingless (Wg), in *Drosophila*, and, its vertebrate homolog, Wnt signalling pathways regulate cell profileration. Wg and Wnt are secreted growth factors which are involved in triggering cellular decisions. The Wg/Wnt ligand binds to Frizzled (Fz) family receptor molecules to initiate a signal transduction cascade involving the cytoplasmic protein Dishevelled (Dvl) (Sussman DJ *et al*). The GenBank accession number for *Dvl-l* cDNA is U10115. The complex illustrated in Figure 3 is present in the cytoplasm of the target cell. Generally APC blocks signalling; however, in the presence of signalling from Wnt, β-catenin is released and interacts with two transcription factors - Lef-1TCF-1 resulting in target gene expression. Target genes of Wnt include En and therefore indirectly HH, c-myc and cyclin D1. It will be appreciated that Notch signalling is also regulated by the Wnt pathway, as Dvl has been found to inhibit Notch signalling.

### Inhibitors

The present invention relates to the use of compounds which inhibit or block (antagonise) Hedgehog signalling. Such compounds may be seen as having the effect of downregulating the expression of Hedgehog. Conveniently such compounds may be referred to herein as inhibitors or antagonists.

The invention contemplates that mutations that result in loss of normal function of the regulators of the Hedgehog signalling pathway or regulators of a pathway which is a target of the Hedgehog signalling pathway in human disease states in which lymphocyte infiltration or failure of a cell cycle checkpoint is involved. Gene therapy to restore such regulatory activity would thus be indicated in treating those disease states Alternatively, it is contemplated that preventing the expression of or inhibiting the activity of such signalling pathways will be useful in treating the disease states. It is contemplated that antisense therapy or gene therapy could be applied to negatively regulate such signalling pathways.

Antagonists for each component of the signalling pathway have been identified. These may be summarised as follows in Table 3:

**Table 3**

| **Component** | **Antagonist** |
|---|---|
| HH | Hip (Chuang and McMahon), Veratrum alkaloids and distal inhibitors of cholesterol biosynthesis (Cooper *et al*) e.g. cyclopamine (Coventry *et al*). |
| Wnt | Frezzled (Leyns *et al*), Cerberus (Bouwmeester *et al*), Gremlin (Hsu *et al*), WIF-1 (Hsieh *et al*) |
| BMP | Noggin (Valenzuela *et al*), Chordin (Sasai *et al*), Cerberus, Gremlin, Xnr-3 |
| Activin | Follistatin (Iemura *et al*) |

HIP (for Hedgehog-interacting protein) is a membrane glycoprotein that binds to at least all three mammalian Hedgehog proteins with an affinity comparable to that of Ptc. HIP appears to attenuate Hedgehog signalling as a result of binding to Hedgehog proteins. Such a negative regulatory feedback loop could also serve to modulate the response to any Hedgehog signal. The GenBank accession number for HIP is AF116865.

Veratrum alkaloids and distal inhibitors of cholesterol biosynthesis have been studied for more than 30 years as potent teratogens capable of inducing cyclopia and other birth defects. It has also been shown that these compounds specifically block the Shh signaling pathway (Cooper *et al*). One example of such a veraturm alkaloid is cyclopamine (11-deoxojervine), a steroid isolated from the desert plant *Veratrum californicum* (Coventry *et al*).

Frezzled is a secreted antagonist of Wnt signalling. Frezzled contains a domain similar to the putative Wnt-binding region of the Frizzled family of transmembrane receptors, but it lacks all the transmembrane domains resulting in a putative secreted Wnt-binding protein. The GenBank accession numbers for the *Xenopus,* mouse and human Frezzled cDNA sequences are U68059, U68058 and U68057, respectively.

Cerberus is a secreted protein and it has been found to be an antagonist of the Wnt and BMP signalling pathways. The GenBank accession number for the *Xenopus* Cerberus cDNA is U64831.

WIF-1 (Wnt-inhibitory factor-1) is a secreted protein which binds to Wnt proteins and inhibits their activities. GenBank accession numbers for WIF-1 are: human, AF122922; mouse, AF122923; *Xenopus,* AF122924; and zebrafish, AF122925.

Noggin and Chordin bind to BMPs thereby preventing activation of their signalling cascade.

Gremlin is a secreted protein and it has been found to be an antagonist of the Wnt and BMP signalling pathways. The GenBank accession numbers for Gremlin cDNA are: *Xenopus*, AF045798; chick, AF045799; human, AF045800; and mouse, AF045801.

Xnr-3 has been found to be an antagonist of BMP signalling pathways.

Follistatin has been found to inhibit others aspects of BMP activity as well as acting as an activin-binding protein.

It will also be appreciated that the antagonist may itself be a component of the Hedgehog signalling pathway. Examples of such antagonists include the negative regulators of HH signalling: Ptc, Cos2 and PKA.

In a particularly preferred embodiment use is made of PKA. PKA has been implicated in the mechanism of Hh signal transduction because it acts to repress Hh target genes in imaginal disc cells that express Ci. Ci action as transcriptional repressor or activator is contingent upon Hedgehog-regulated, PKA-dependent proteolytic processing.

Cyclic AMP (cAMP) is a nucleotide that is generated from ATP in response to hormonal stimulation of cell-surface receptors. cAMP acts as a signaling molecule by activating A-kinase; it is hydrolyzed to AMP by phosphodiesterase (PDE). cAMP levels affect cubitus cleavage and TGF-β levels. Specifically, when cAMP levels increase, TGF-β levels decrease and this will affect fibrosis, for example. In another embodiment of the invention use is made of cAMP modifiers. Such modifiers include PDE inhibitors, and beta-agonists such as the beta-adrenergic agonist. For example, it has been found that ptc 1 transcription can be induced by agents activating the cAMP signal transduction pathway.

Antisense nucleic acids (preferably 10 to 20 base pair oligonucleotides) capable of specifically binding to expression control sequences or RNA are introduced into cells (*e*.*g*., by a viral vector or colloidal dispersion system such as a liposome). The antisense nucleic acid binds to the target sequence in the cell and prevents transcription or translation of the target sequence. Phosphothioate and methylphosphate antisense oligonucleotides are specifically contemplated for therapeutic use by the invention. The antisense oligonucleotides may be further modified by poly-L-lysine, transferrin polylysine, or cholesterol moieties at their 5' end.

Also comprehended by the present invention are antibody products (*e.g.*, monoclonal and polyclonal antibodies, single chain antibodies, chimeric antibodies, CDR-grafted antibodies and antigen-binding fragments thereof) and other binding proteins (such as those identified in the assays above). Binding proteins can be developed using isolated natural or recombinant enzymes. The binding proteins are useful, in turn, for purifying recombinant and naturally occurring enzymes and identifying cells producing such enzymes. Assays for the detection and quantification of proteins in cells and in fluids may involve a single antibody substance or multiple antibody substances in a "sandwich" assay format to determine cytological analysis of HH protein levels. The binding proteins are also manifestly useful in modulating (*i*.*e*. blocking, inhibiting, or stimulating) interactions.

Antibodies may be generated by administering polypeptides or epitope-containing fragments to an animal, usually a rabbit, using routine protocols. Examples of such techniques include those in Kohler and Milstein.

More generally, the antagonist may be selected from polypeptides and fragments thereof, linear peptides, cyclic peptides, synthetic and natural compounds including low molecular weight organic or inorganic compounds. The antagonist may be derived from a biological material such as a component of extracellular matrix.

Polypeptide substances, such as Noggin or Chordin, may be purified from mammalian cells, obtained by recombinant expression in suitable host cells or obtained commercially. Alternatively, nucleic acid constructs encoding the polypeptides may be introduced by transfection using standard techniques or viral infection/transduction.

Inhibitors for use according to the present invention may be conveniently identified using a convenient screening procedure.

One assay for identifying such inhibitors may involve immobilizing a component of the relevant pathway, e.g. HH, or a test protein, detectably labelling the nonimmobilized binding partner, incubating the binding partners together and determining the amount of label bound. Bound label indicates that the test protein interacts with the component.

Another type of assay for identifying inhibitors involves immobilizing a component of the pathway, e.g. HH, or a fragment thereof on a solid support coated (or impregnated with) a fluorescent agent, labelling a test protein with a compound capable of exciting the fluorescent agent, contacting the immobilized component with the labelled test protein, detecting light emission by the fluorescent agent, and identifying interacting proteins as test proteins which result in the emission of light by the fluorescent agent. Alternatively, the putative interacting protein may be immobilized and the component may be labelled in the assay.

Moreover, such assays for identifying inhibitors may involve: transforming or transfecting appropriate host cells with a DNA construct comprising a reporter gene under the control of a promoter regulated by a transcription factor having a DNA-binding domain and an activating domain; expressing in the host cells a first hybrid DNA sequence encoding a first fusion of part or all of a component of the pathway, e.g. HH, Wnt or BMP, and the DNA binding domain or the activating domain of the transcription factor; expressing in the host cells a second hybrid DNA sequence encoding part or all of a protein that interacts with said component and the DNA binding domain or activating domain of the transcription factor which is not incorporated in the first fusion; evaluating the effect of a test compound on the interaction between said component and the interacting protein by detecting binding of the interacting protein to said component in a particular host cell by measuring the production of reporter gene product in the host cell in the presence or absence of the test compound; and identifying modulating compounds as those test compounds altering production of the reported gene product in comparison to production of the reporter gene product in the absence of the modulating compound. Presently preferred for use in the assay are a *lexA* promoter to drive expression of the reporter gene, the *lacZ* reporter gene, a transcription factor comprising the *lexA* DNA binding domain and the GAL4 transactivation domain, and yeast host cells.

In a particular embodiment described in relation to Hedgehog signalling the appropriate host cell is transformed or transfected with a DNA construct comprising a reporter gene under the control of the Ptc promoter; expressing in said cells a DNA sequence encoding Hedgehog; evaluating the effect of a test compound on the interaction between HH and the Ptc promoter in a particular host cell by measuring the production of reporter gene product in the host cell in the absence and presence of the test compound; and identifying inhibitors as those test compounds reducing the production of the reporter gene product in comparison to production of the reporter gene product in the absence of the test compound.

Combinatorial libraries, peptide and peptide mimetics, defined chemical entities, oligonucleotides, and natural product libraries may be screened for activity as inhibitors in such assays.

The present invention also relates to the use of derivatives, variants, fragments, analogues, homologues and mimetics of the inhibitors mentioned above, including those identifiable using the assay procedures.

The term "derivative" as used herein in relation to the polypeptides of the present invention includes any substitution of, variation of, modification of, replacement of, deletion of, or addition of one for more) amino acid residues from or to the sequence providing that the resultant protein etc., possesses the capability to antagonise the action of the signalling pathway.

The term "variant" as used herein in relation to the polypeptides of the present invention includes any substitution of, variation of, modification of, replacement of, deletion of, or addition of one (or more) amino acid residues from or to the sequence providing that the resultant protein etc., possesses the capability to antagonise the action of the signalling pathway.

The term "fragment" as used herein in relation to the polypeptides of the present invention includes a variant polypeptide which has an amino acid sequence that is entirely the same as part but not all of the amino acid sequence of the aforementioned polypeptide and possesses the capability to antagonise the action of the signalling pathway.

The term "analogue" as used herein in relation to the polypeptides of the present invention includes any peptidomimetic, i.e. a chemical compound that possess the capability to antagonise the action of the signalling pathway in a similar manner to the parent polypeptide.

The term "homologue" as used herein in relation to the polypeptides of the present invention includes a polypeptide which has the same evolutionary origin as the subject polypeptide providing that it possesses the capability to antagonise the action of the signalling pathway.

The term "mimetic" as used herein in relation to the inhibitors of the present invention includes a compound which also possesses the capability to antagonise the action of the signalling pathway in a similar manner to the parent compound.

More particularly, the term "homologue" covers identity with respect to structure and/or function providing the expression product of the resultant nucleotide sequence has the inhibitory activity. With respect to sequence identity (i.e. similarity), preferably there is at least 75%, more preferably at least 85%, more preferably at least 90% sequence identity. More preferably there is at least 95%, more preferably at least 98%, sequence identity. These terms also encompass allelic variations of the sequences.

Sequence identity with respect to the sequences can be determined by a simple "eyeball" comparison (i.e. a strict comparison) of any one or more of the sequences with another sequence to see if that other sequence has, for example, at least 75% sequence identity to the sequence(s).

Relative sequence identity can also be determined by commercially available computer programs that can calculate % identity between two or more sequences using any suitable algorithm for determining identity, using for example default parameters. A typical example of such a computer program is CLUSTAL. Advantageously, the BLAST algorithm is employed, with parameters set to default values. The BLAST algorithm is described in detail at http://www.ncbi.nih.gov/BLAST/blast_help.html, which is incorporated herein by reference. The search parameters are defined as follows, can be advantageously set to the defined default parameters.

Advantageously, "substantial identity" when assessed by BLAST equates to sequences which match with an EXPECT value of at least about 7, preferably at least about 9 and most preferably 10 or more. The default threshold for EXPECT in BLAST searching is usually 10.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul (see http://www.ncbi.nih.gov/BLAST/blast_help.html) with a few enhancements. The BLAST programs were tailored for sequence similarity searching, for example to identify homologues to a query sequence. For a discussion of basic issues in similarity searching of sequence databases, see Altschul *et al* (1994) Nature Genetics 6:119-129.

The five BLAST programs available at http://www.ncbi.nlm.nih.gov perform the following tasks:
**blastp -** compares an amino acid query sequence against a protein sequence database.
**blastn -** compares a nucleotide query sequence against a nucleotide sequence database.
**blastx -** compares the six-frame conceptual translation products of a nucleotide query sequence (both strands) against a protein sequence database.
**tblastn** - compares a protein query sequence against a nucleotide sequence database dynamically translated in all six reading frames (both strands).
**tblastx -** compares the six-frame translations of a nucleotide query sequence against the six-frame translations of a nucleotide sequence database.

### BLAST uses the following search parameters:

HISTOGRAM - Display a histogram of scores for each search; default is yes. (See parameter H in the BLAST Manual).

DESCRIPTIONS - Restricts the number of short descriptions of matching sequences reported to the number specified; default limit is 100 descriptions. (See parameter V in the manual page).

EXPECT - The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).

CUTOFF - Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.

ALIGNMENTS - Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).

MATRIX - Specify an alternate scoring matrix for BLASTP, BLASTX, TBLASTN and TBLASTX. The default matrix is BLOSUM62 (Henikoff & Henikoff, 1992). The valid alternative choices include: PAM40, PAM120, PAM250 and IDENTITY. No alternate scoring matrices are available for BLASTN; specifying the MATRIX directive in BLASTN requests returns an error response.

STRAND - Restrict a TBLASTN search to just the top or bottom strand of the database sequences; or restrict a BLASTN, BLASTX or TBLASTX search to just reading frames on the top or bottom strand of the query sequence.

FILTER - Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States (1993) Computers and Chemistry 17:191-201, or, for BLASTN, by the DUST program of Tatusov and Lipman (see http://www.ncbi.nlm.nih.gov). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "NNNNNNNNNNNNN") and the letter "X" in protein sequences (e.g., "XXXXXXXXX").

Filtering is only applied to the query sequence (or its translation products), not to database sequences. Default filtering is DUST for BLASTN, SEG for other programs.
It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi - Causes NCBI gi identifiers to be shown in the output, in addition to the accession and/or locus name.

Most preferably, sequence comparisons are conducted using the simple BLAST search algorithm provided at http://www.ncbi.nlm.nih.gov/BLAST.

Other computer program methods to determine identify and similarity between the two sequences include but are not limited to the GCG program package (Devereux *et al* 1984 Nucleic Acids Research 12: 387) and FASTA (Atschul *et al* 1990 J Molec Biol 403-410).

In some aspects of the present invention, no gap penalties are used when determining sequence identity.

The present invention also encompasses use of nucleotide sequences that are complementary to the sequences presented herein, or any fragment or derivative thereof. If the sequence is complementary to a fragment thereof then that sequence can be used as a probe to identify similar promoter sequences in other organisms.

The present invention also encompasses use of nucleotide sequences that are capable of hybridising to the sequences presented herein, or any fragment or derivative thereof.

Hybridization means a "process by which a strand of nucleic acid joins with a complementary strand through base pairing" (Coombs J (1994) Dictionary of Biotechnology, Stockton Press, New York NY) as well as the process of amplification as carried out in polymerase chain reaction technologies as described in Dieffenbach CW and GS Dveksler (1995, PCR Primer, a Laboratory Manual, Cold Spring Harbor Press, Plainview NY).

Also included within the scope of the present invention are use of nucleotide sequences that are capable of hybridizing to the nucleotide sequences presented herein under conditions of intermediate to maximal stringency. Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical nucleotide sequences while an intermediate (or low) stringency hybridization can be used to identity or detect similar or related nucleotide sequences.

In a preferred aspect, the present invention covers use of nucleotide sequences that can hybridise to the nucleotide sequences of the present invention under stringent conditions (e.g. 65°C and 0.1xSSC).

The present invention also encompasses use of nucleotide sequences that are capable of hybridising to the sequences that are complementary to the sequences presented herein, or any fragment or derivative thereof. Likewise, the present invention encompasses use of nucleotide sequences that are complementary to sequences that are capable of hybridising to the sequence of the present invention. These types of nucleotide sequences are examples of variant nucleotide sequences.

In this respect, the term "variant" encompasses sequences that are complementary to sequences that are capable of hydridising to the nucleotide sequences presented herein. Preferably, however, the term "variant" encompasses sequences that are complementary to sequences that are capable of hydridising under stringent conditions (eg. 65°C and 0.1xSSC {1xSSC = 0.15 M NaCl, 0.015 Na₃ citrate pH 7.0}) to the nucleotide sequences presented herein.

### Transgenic Animals

Transgenic animals which are capable of expressing or overexpressing at least one antagonist useful in the present invention are described. Preferably the animal expresses or overexpresses HIP, Frezzled-1, Noggin (Ngg) and/orWIF-1.

Transgenic animals may be capable of expressing or overexpressing at least one polypeptide which is a component of the Hedgehog signalling pathway or a component of a pathway which is a target of the Hedgehog signalling pathway, such as the Wnt or BMP signalling pathway. Preferably the animal expresses or overexpresses HH (more preferably Shh), and/or Dvl-1.

The transgenic animal is typically a vertebrate, more preferably a rodent, such as a rat or a mouse, but also includes other mammals such as human, goat, pig or cow etc.

Such transgenic animals are useful as animal models of disease and in screening assays for new useful compounds. By specifically expressing one or more polypeptides, as defined above, the effect of such polypeptides on the development of disease can be studied. Furthermore, therapies including gene therapy and various drugs can be tested on transgenic animals. Methods for the production of transgenic animals are known in the art. For example, there are several possible routes for the introduction of genes into embryos. These include (i) direct transfection or retroviral infection of embryonic stem cells followed by introduction of these cells into an embryo at the blastocyst stage of development; (ii) retroviral infection of early embryos; and (iii) direct microinjection of DNA into zygotes or early embryo cells.

Stable cell lines useful as disease models for testing or treatment are also described.

A stable cell line will contain a recombinant gene or genes, also known herein as a transgene, encoding one or more inhibitors or components of a Hedgehog signalling pathway.

Preferably the transgene is HH (more preferably Shh), HIP, WIF-1, Fzb-1, Ngg and/or Dvl-1. A cell line containing a transgene, as described herein, is made by introducing the transgene into a selected cell line according to one of several procedures known in the art for introducing a foreign gene into a cell.

As also described below, the sequences encoding the inhibitors and components of signalling pathways, as described herein, are operably linked to control sequences, including promoters/enhancers and other expression regulation signals.

The promoter is typically selected from promoters which are functional in mammalian cells, although prokaryotic promoters and promoters functional in other eukaryotic cells may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of a-actin, b-actin, tubulin) or, alternatively, a tissue-specific manner (such as promoters of the genes for pyruvate kinase). Tissue-specific promoters specific for lymptocytes, dendritic cells, skin, brain cells and epithelial cells within the eye are particularly preferred, for example the CD2, CD11c, keratin 14, Wnt-1 and Rhodopsin promoters respectively. Preferably the epithelial cell promoter SPC is used. They may also be promoters that respond to specific stimuli, for example promoters that bind steroid hormone receptors. Viral promoters may also be used, for example the Moloney murine leukaemia virus long terminal repeat (MMLV LTR) promoter, the rous sarcoma virus (RSV) LTR promoter or the human cytomegalovirus (CMV) IE promoter.

It may also be advantageous for the promoters to be inducible so that the levels of expression of the heterologous gene can be regulated during the life-time of the cell. Inducible means that the levels of expression obtained using the promoter can be regulated.

In addition, any of these promoters may be modified by the addition of further regulatory sequences, for example enhancer sequences. Chimeric promoters may also be used comprising sequence elements from two or more different promoters described above.

### Therapeutic Uses

As previously mentioned, in many tissues, such as lung and kidney, inflammation may lead to chronic diseases. For example, chronic lung disease involve tissue remodelling in the presence of inflammatory cells, examples are emphysema and interstitial lung disease (ILD). The disease tissue is associated with epithelial cell hyperplasia leading to fibrosis and scarring. There is an accompanying mononuclear cell infiltration at local sites of inflammation and the induction of immune responses reactive with self antigens. Similar pathology is also observed in the chronic rejection of graft tissue, including transplanted organs.

Hedgehog is important in regulating growth and differentiation of epithelial cells. It has a role in the formation of notochord, limb, gut, lung, skin etc. Branching morphogenesis occurs through induction of Wnt and BMP growth factors. It binds to its receptor Ptc and Smo. Mutations can lead to the human autosomal disorder Nevoid basal cell carcinoms syndrome (NBCCS) which is characterised by developmental abnormalities and a high predisposition for various forms of cancer mainly the very common basal cell carcinomas (BCC).

BMPs are members of the TGF-β superfamily. Whereas a role for TGF-β1 in mediating lung fibrosis is well established, previously BMP-4 has only been implicated in fibrotic disease of bone.

We now provide a medicament for the treatment of epithelial cell hyperplasia and fibrosis particularly in the lung and kidney. More particularly diseases which may be treated include adult respiratory distress syndrome; chronic obstructive airway disorders/ chronic obstructive pulmonary disease including asthma, emphysema and chronic bronchitis; atelectasis; occupational lung disease including silicosis; hypersensitivity diseases of the lung including hypersensitivity pneomonitis; idiopathic interstitial lung diseases including idiopathic pulmonary fibrosis, usual interstitial pneumonia, desquamative interstitial pneumonia and acute interstitial pneumonia; and pleural fibrosis. Further details on such conditions and those given below may be found in The Merck Manual (17th Edition), published by Merck Research Laboratories, N.J., USA.

The present invention is also useful in treating immune disorders such as autoimmune diseases or graft rejection such as allograft rejection.

Examples of disorders that may be treated include a group commonly called autoimmune diseases. The spectrum of autoimmune disorders ranges from organ specific diseases (such as thyroiditis, insulitis, multiple sclerosis, iridocyclitis, uveitis, orchitis, hepatitis, Addison's disease, myasthenia gravis) to systemic illnesses such as rheumatoid arthritis or lupus erythematosus. Other disorders include immune hyperreactivity, such as allergic reactions.

In more detail: Organ-specific autoimmune diseases include multiple sclerosis, insulin dependent diabetes mellitus, several forms of anemia (aplastic, hemolytic), autoimmune hepatitis, thyroiditis, insulitis, iridocyclitis, skleritis, uveitis, orchitis, myasthenia gravis, idiopathic thrombocytopenic purpura, inflammatory bowel diseases (Crohn's disease, ulcerative colitis).

Systemic autoimmune diseases include: rheumatoid arthritis, juvenile arthritis, scleroderma and systemic sclerosis, sjogren's syndrom, undifferentiated connective tissue syndrome, antiphospholipid syndrome, different forms of vasculitis (polyarteritis nodosa, allergic granulomatosis and angiitis. Wegner's granulomatosis, Kawasaki disease, hypersensitivity vasculitis, Henoch-Schoenlein purpura, Behcet's Syndrome, Takayasu arteritis, Giant cell arteritis, Thrombangiitis obliterans), lupus erythematosus, polymyalgia rheumatica, essentiell (mixed) cryoglobulinemia, Psoriasis vulgaris and psoriatic arthritis, diffus fasciitis with or without eosinophilia, polymyositis and other idiopathic inflammatory myopathies, relapsing panniculitis, relapsing polychondritis, lymphomatoid granulomatosis, erythema nodosum, ankylosing spondylitis, Reiter's syndrome, different forms of inflammatory dermatitis.

A more extensive list of disorders includes: unwanted immune reactions and inflammation including arthritis, including rheumatoid arthritis, inflammation associated with hypersensitivity, allergic reactions, asthma, systemic lupus erythematosus, collagen diseases and other autoimmune diseases, inflammation associated with atherosclerosis, arteriosclerosis, atherosclerotic heart disease, reperfusion injury, cardiac arrest, myocardial infarction, vascular inflammatory disorders, respiratory distress syndrome or other cardiopulmonary diseases, inflammation associated with peptic ulcer, ulcerative colitis and other diseases of the gastrointestinal tract, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimo-orchitis, infertility, orchidal trauma or other immune-related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, e.g. following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery or organ, inflammatory and/or immune complications and side effects of gene therapy, e.g. due to infection with a viral carrier, or inflammation associated with AIDS, to suppress or inhibit a humoral and/or cellular immune response, to treat or ameliorate monocyte or leukocyte proliferative diseases, e.g. leukaemia, by reducing the amount of monocytes or lymphocytes, for the prevention and/or treatment of graft rejection in cases of transplantation of natural or artificial cells, tissue and organs such as cornea, bone marrow, organs, lenses, pacemakers, natural or artificial skin tissue.

We have now found that the use of antagonists of Hedgehog signalling may prevent and/or promote regression of the above-mentioned diseases.

### Vectors, host cells, expression

Vectors which comprise a polynucleotide useful in the present invention are described, as well as, host cells which are genetically engineered with such vectors and the production of polypeptides useful in the present invention by such techniques.

For recombinant production, host cells can be genetically engineered to incorporate expression systems or polynucleotides of the invention. Introduction of a polynucleotide into the host cell can be effected by methods described in many standard laboratory manuals, such as Davis *et al* and Sambrook *et al,* such as calcium phosphate transfection. DEAE-dextran mediated transfection, transvection, microinjection, cationic lipid-mediated transfection, electroporation, transduction, scrape loading, ballistic introduction and infection.

Representative examples of appropriate hosts include bacterial cells, such as streptococci, staphylococci, *E*. *Coli*, streptomyces and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, 293 and Bowes melanoma cells; and plant cells.

A great variety of expression systems can be used to produce a polypeptide useful in the present invention. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques, such as, for example, those set forth in Sambrook *et al.*

For secretion of the translated protein into the lumen of the endoplasmic reticulum, into the periplasmic space or into the extracellular environment, appropriate secretion signals may be incorporated into the expressed polypeptide. These signals may be endogenous to the polypeptide or they may be heterologous signals.

Polypeptides of the invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding protein may be employed to regenerate active conformation when the polypeptide is denatured during isolation and/or purification.

### Methods of Delivery

In the present invention the polynucleotide may be delivered to a target cell population, either *ex vivo* or *in vivo*, by any suitable Gene Delivery Vehicle.

This includes but is not restricted to, DNA, formulated in lipid or protein complexes or administered as naked DNA via injection or biolistic delivery, viruses such as retroviruses, adenoviruses, herpes viruses, vaccinia viruses, adeno associated viruses. The GDV can be designed by a person ordinarily skilled in the art of recombinant DNA technology and gene expression to express the fusion protein at appropriate levels and with the cellular specificity demanded by a particular application.

As it is well known in the art, a vector is a tool that allows or facilitates the transfer of an entity from one environment to another. In accordance with the present invention, and by way of example, some vectors used in recombinant DNA techniques allow entities, such as a segment of DNA (such as a heterologous DNA segment, such as a heterologous cDNA segment), to be transferred into a target cell. Optionally, once within the target cell, the vector may then serve to maintain the heterologous DNA within the cell or may act as a unit of DNA replication. Examples of vectors used in recombinant DNA techniques include plasmids, chromosomes, artificial chromosomes or viruses.

The vector can be delivered by viral or non-viral techniques.

Non-viral delivery systems include but are not limited to DNA transfection methods. Here, transfection includes a process using a non-viral vector to deliver a gene to a target mammalian cell.

Typical transfection methods include electroporation, DNA biolistics, lipid-mediated transfection, compacted DNA-mediated transfection, liposomes, immunoliposomes, lipofectin, cationic agent-mediated, cationic facial amphiphiles (CFAs) (Nature Biotechnology 1996 14; 556), multivalent cations such as spermine, cationic lipids or polylysine, 1, 2,-bis (oleoyloxy)-3-(trimethylammonio) propane (DOTAP)-cholesterol complexes (Wolff and Trubetskoy 1998 Nature Biotechnology 16: 421) and combinations thereof.

Viral delivery systems include but are not limited to adenovirus vector, an adeno-associated viral (AAV) vector, a herpes viral vector, a retroviral vector, a lentiviral vector or a baculoviral vector.

Examples of retroviruses include but are not limited to: murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV).

A detailed list of retroviruses may be found in Coffin *et al* ("Retroviruses" 1997 Cold Spring Harbour Laboratory Press Eds: JM Coffin, SM Hughes, HE Varmus pp 758-763).

Adenoviruses and adeno-associated viruses which have good specificity for epithelial cells are particularly preferred.

Other examples of vectors include *ex vivo* delivery systems, which include but are not limited to DNA transfection methods such as electroporation, DNA biolistics, lipid-mediated transfection, compacted DNA-mediated transfection.

Thus, nucleic acid vectors as described above may be capable of delivery preferentially to the target cell. For example in the case of a retroviral vector, the retroviral envelope protein may be capable of directing the vector to a particular cell type or cell types. For that purpose, the envelope protein may be a modified envelope protein adapted to have a specific targeting ability, or it may be a selected envelope protein derived from a different viral or retroviral source and having the desired targeting ability.

Preferably, the nucleic acid in a vector is operatively linked to an expression control sequence capable of causing preferential expression of the fusion protein in the target cell. The expression control sequence may be for example a promotor or enhancer which is preferentially active in certain cell types including the target cell, or a promotor or enhancer which is preferentially active under certain conditions.

The term "promoter" is used in the normal sense of the art, e.g. an RNA polymerase binding site in the Jacob-Monod theory of gene expression.

The term "enhancer" includes a DNA sequence which binds to other protein components of the transcription initiation complex and thus facilitates the initiation of transcription directed by its associated promoter.

Preferably the promoters of the present invention are tissue specific. That is, they are capable of driving transcription of a nucleic acid in one tissue while remaining largely "silent" in other tissue types. A particularly preferred promoter is the epithelial cell promoter.

The term "tissue specific" means a promoter which is not restricted in activity to a single tissue type but which nevertheless shows selectivity in that they may be active in one group of tissues and less active or silent in another group.

### Administration

Compounds capable of affecting a component of the Hedgehog family signalling pathway for use in therapy are typically formulated for administration to patients with a pharmaceutically acceptable carrier or diluent to produce a pharmaceutical composition. The formulation will depend upon the nature of the compound identified and the route of administration but typically they can be formulated for topical, parenteral, intramuscular, intravenous, intra-peritoneal, intranasal inhalation, lung inhalation, intradermal or intra-articular administration. The compound may be used in an injectable form. It may therefore be mixed with any vehicle which is pharmaceutically acceptable for an injectable formulation, preferably for a direct injection at the site to be treated, although it may be administered systemically.

The pharmaceutically acceptable carrier or diluent may be, for example, sterile isotonic saline solutions, or other isotonic solutions such as phosphate-buffered saline. The compounds of the present invention may be admixed with any suitable binder(s), lubricant(s), suspending agent(s), coating agent(s), solubilising agent(s). It is also preferred to formulate the compound in an orally active form.

In general, a therapeutically effective daily oral or intravenous dose of the compounds of the invention, including compounds of formula (1) and their salts, is likely to range from 0.01 to 50 mg/kg body weight of the subject to be treated, preferably 0.1 to 20 mg/kg. The compounds of the formula (I) and their salts may also be administered by intravenous infusion, at a dose which is likely to range from 0.001-10 mg/kg/hr.

Tablets or capsules of the compounds may be administered singly or two or more at a time, as appropriate. It is also possible to administer the compounds in sustained release formulations.

Typically, the physician will determine the actual dosage which will be most suitable for an individual patient and it will vary with the age, weight and response of the particular patient. The above dosages are exemplary of the average case. There can, of course, be individual instances where higher or lower dosage ranges are merited, and such are within the scope of this invention.

Alternatively, the compounds of the invention can be administered by inhalation or in the form of a suppository or pessary, or they may be applied topically in the form of a lotion, solution, cream, ointment or dusting powder. An alternative means of transdermal administration is by use of a skin patch. For example, they can be incorporated into a cream consisting of an aqueous emulsion of polyethylene glycols or liquid paraffin. They can also be incorporated, at a concentration of between 1 and 10% by weight, into an ointment consisting of a white wax or white soft paraffin base together with such stabilisers and preservatives as may be required.

For some applications, preferably the compositions are to be administered orally in the form of tablets containing excipients such as starch or lactose, or in capsules or ovules either alone or in admixture with excipients, or in the form of elixirs, solutions or suspensions containing flavouring or colouring agents.

The compositions (as well as the compounds alone) can also be injected parenterally, for example intracavernosally, intravenously, intramuscularly or subcutaneously. In this case, the compositions will comprise a suitable carrier or diluent.

For parenteral administration, the compositions are best used in the form of a sterile aqueous solution which may contain other substances, for example enough salts or monosaccharides to make the solution isotonic with blood.

For buccal or sublingual administration the compositions may be administered in the form of tablets or lozenges which can be formulated in a conventional manner.

For oral, parenteral, buccal and sublingual administration to subjects (such as patients), the daily dosage level of the compounds of the present invention and their pharmaceutically acceptable salts and solvates may typically be from 10 to 500 mg (in single or divided doses). Thus, and by way of example, tablets or capsules may contain from 5 to 100 mg of active compound for administration singly, or two or more at a time, as appropriate. As indicated above, the physician will determine the actual dosage which will be most suitable for an individual patient and it will vary with the age, weight and response of the particular patient. It is to be noted that whilst the above-mentioned dosages are exemplary of the average case there can, of course, be individual instances where higher or lower dosage ranges are merited and such dose ranges are within the scope of this invention.

The routes of administration and dosages described are intended only as a guide since a skilled practitioner will be able to determine readily the optimum route of administration and dosage for any particular patient depending on, for example, the age, weight and condition of the patient.

The term treatment or therapy as used herein should be taken to encompass diagnostic and prophylatic applications.

The use of the present invention includes both human and veterinary applications.

As used herein the terms protein and polypeptide and peptide may be assumed to be synonymous, protein merely being used in a general sense to indicate a relatively longer amino acid sequence than that present in a polypeptide, and polypetide merely being used in a general sense to indicate a relatively longer amino acid sequence than that present in a peptide. Generally for ease of reference only we will simply refer to the term polypeptide.

The invention will now be described in further detail with reference to the following non-limiting examples:

### Example 1 : Construction of SPC-Shh expression plasmid

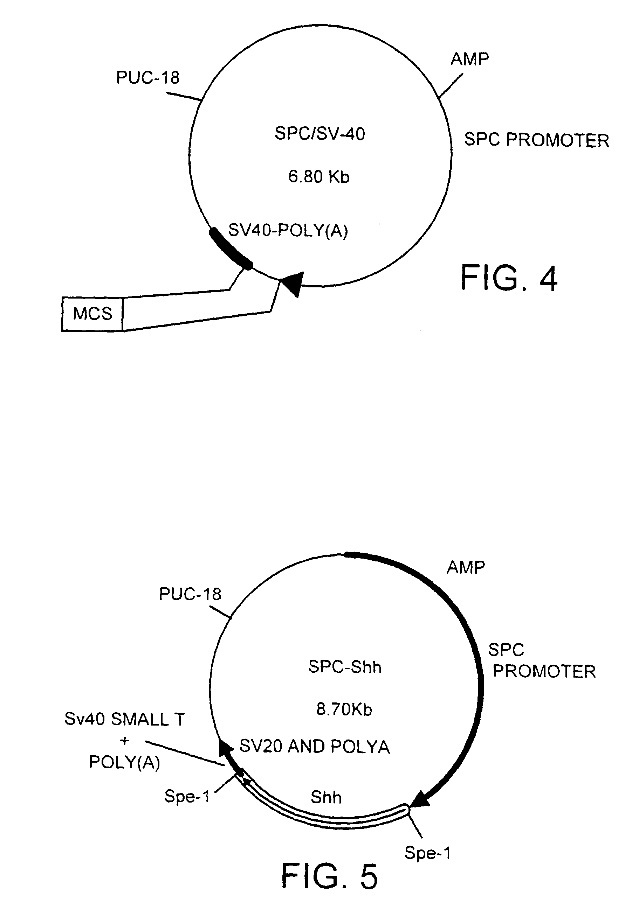

The SPC-mouse Shh vector shown in Figure 5 was constructed from a parent expression vector that was made as follows. The parent vector shown in Figure 4 contains a pUC18 backbone with an ampicillin resistance gene, a 3.7 kb sequence containing the human SPC promoter region, a multiple cloning site (MCS), an SV40 small T intron and a 0.4 kb sequence containing a poly(A) addition site and with stop codons in all three reading frame (Korfhagen *et al;* Development 1997) .The cDNA sequence encoding the mouse Shh was cloned into the MCS.

Additional vectors including SPC-HIP, SPC-WIF-1, SPC-Dvl-1 were made by cloning murine cDNAs for HIP, WIF-1, Dvl-1 into the MCS of the SPC expression vector.

### Example 2 : Increased expression of Ptc in the lung epithelial cells from human patients with idiopathic fibrosing alveolitis (IFA also known as CFA) and in a murine model of interstitial pulmonary fibrosis (IPF).

### Fig. 6C

BALB/c mice were treated intratracheally with 50 µg of FITC disolved in physiological buffered saline (PBS). Three months later mice were sacrificed and lungs removed and fixed in 4% buffered formalin and embedded in paraffin. 5 µm sections of lung tissue were placed onto TESPA coated slides and the expression of Ptc gene expression was examined by anti-sense RNA in situ hybridization (ISH).

Sections were hybridized with digoxigenin antisense RNA probes specific for murine Ptc1 at 65°C . The bound probe was detected by alkaline phosphatase conjugated goat anti-digoxigenin Fab and sections were developed using NBT and BCIP as the substrate. We observed increased expression of Ptc in lung epithelial cells in the murine model of IPF. Expression of Ptc was restricted only to those areas showing damage. Increased Ptc expression was noted within 24 hours of i.t. FITC and was maintained for at least 6 months.

### Fig. 6 A & B

Paraffin embedded archive lung tissue from human patients diagnosed with IFA or control patients with healthy lung tissue were sectioned at 5 µm and placed onto TESPA coated slides. The slides were then analysed for Ptc gene expression by anti-sense RNA in situ hybridization (see above). We observed increased expression of Ptc in lung epithelial cells.

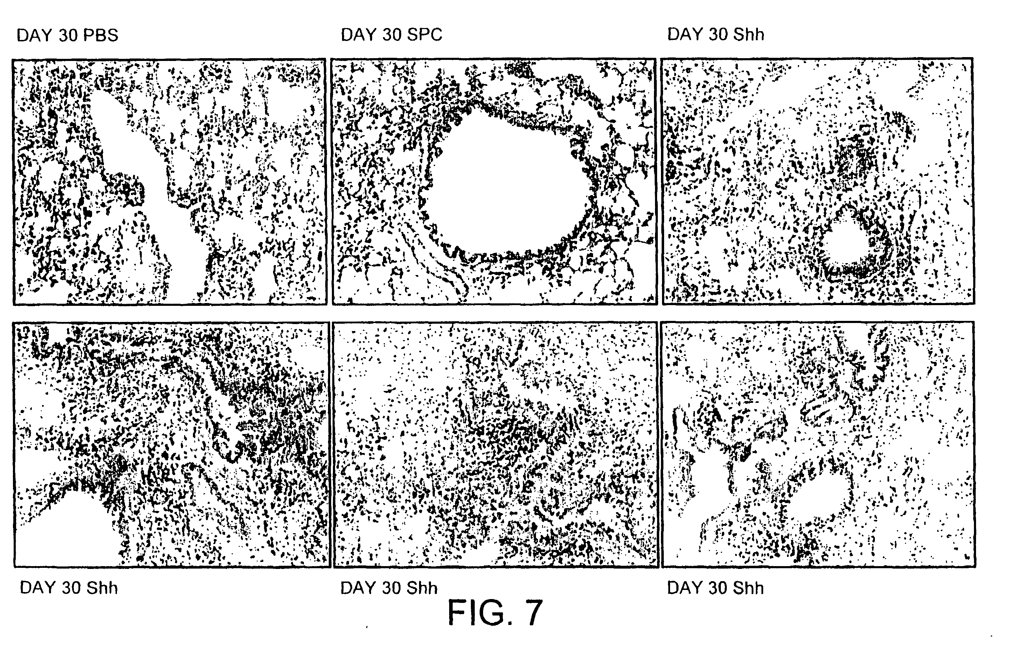

### Example 3 : Overexpression of Shh leads to epithelial cell hyperplasia and lung fibrosis. Figures 7-9

BALB/c mice were injected i.t. with either (i) saline alone, (ii) 20 µg of SPC plasmid dissolved in saline, or (iii) 20 µg of SPC-Shh plasmid DNA dissolved in saline on day 0 and day 5. The SPC plasmid provides tissue-specific expression of a desired gene as it contains the promoter sequence from the lung epithelial cell-specific surfactant protein C (i.e. SPC). Mice were sacificed at day 12 and day 35 where upon the lungs were removed and placed into 4% buffered formalin.

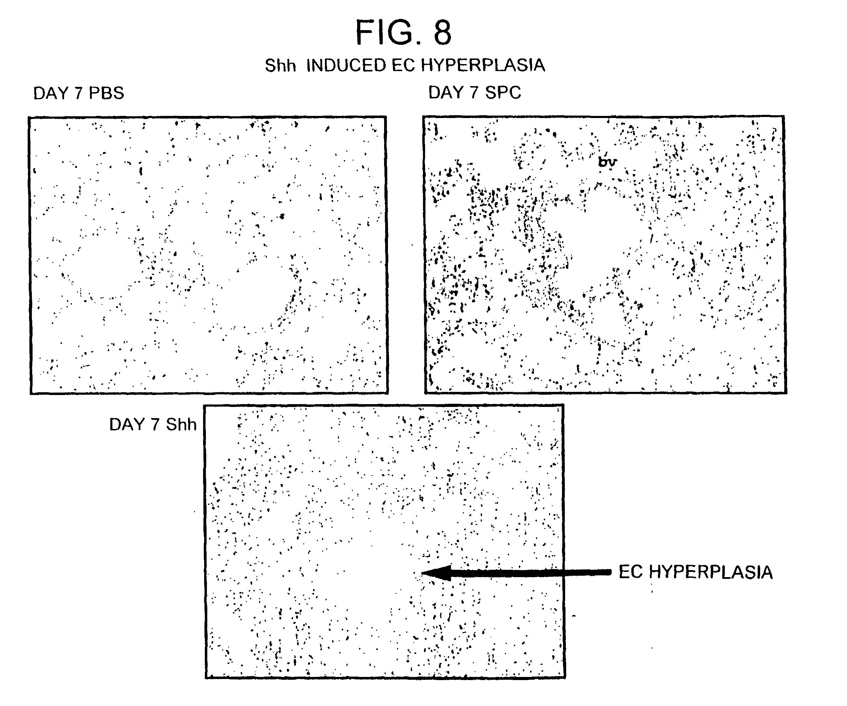

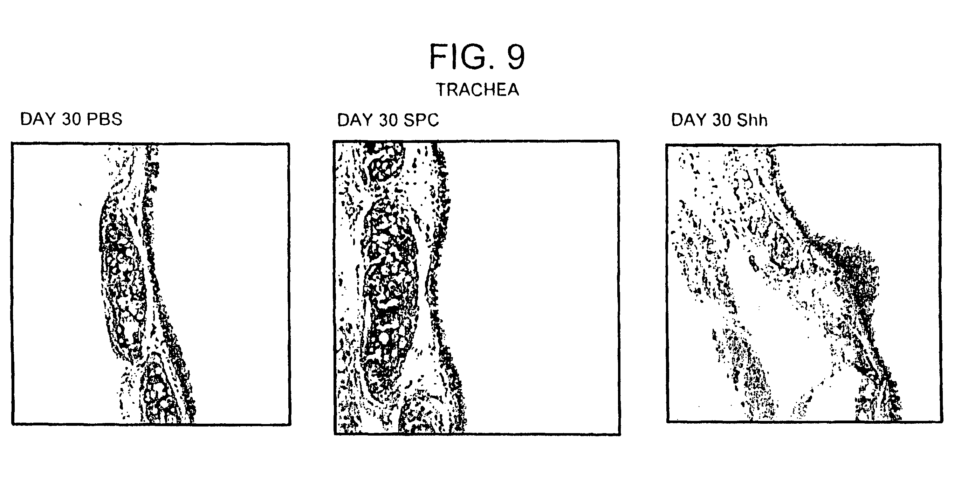

5 µm sections of lung tissue (Fig 7, 8) or trachea (Fig. 9) from each group at each of the two time points were placed onto poly-L-Lysine coated slides and stained using the haematoxylin and eosin (H & E) histochemical stain.
The groups contained:
Day 12 PBS (2 mice), SPC (2 mice) and SPC-shh (2 mice)
Day 35 PBS (3 mice) SPC (3 mice) and SPC-shh (3 mice)

Slides from the day 35 treatment group were further analysed for collagen production using a Masons-trichrome histochemical stain. This type of stain is routinely used to stain for collagen fibres in tissue samples which turn green. Increased levels of collagen staining could be identified in lung tissue from SPC-Shh treated mice when compared to controls.

Slides from the day 35 treatment group were also analysed for potential goblet cell hyperplasia using the periodic acid-schiff (PAS) histochemical stain. Using this stain, cells which generate mucins stain an intense pink colour while epithelium stains light blue. We observed an increase in the number of goblet cells in the lung tissue of SPC-Shh mice (i.e. pink cells) and increased mucous secretion into the airways. The increased goblet cell numbers cells were observed only in those airways showing signs of epithelia hyperplasia. Normal airways in the SPC-Shh mice were equivalent to those in the control mice.

Slides from Day 12 and Day 35 treatment groups were stained for a proliferation index marker, Ki-67, to provide evidence that the lung epithelia of SPC-shh mice were actively proliferating. Slides from the three treatment groups were stained with an antibody specific for the Ki-67 antigen which marks cells in the S-phase of the cell cycle. There was an increase in the number of Ki-67 +ve cells in the lung epithelium of SPC-Shh treated mice when compared to the epithelium of control mice

### Example 4 : Epithelial cells express high levels of Shh following FITC damage

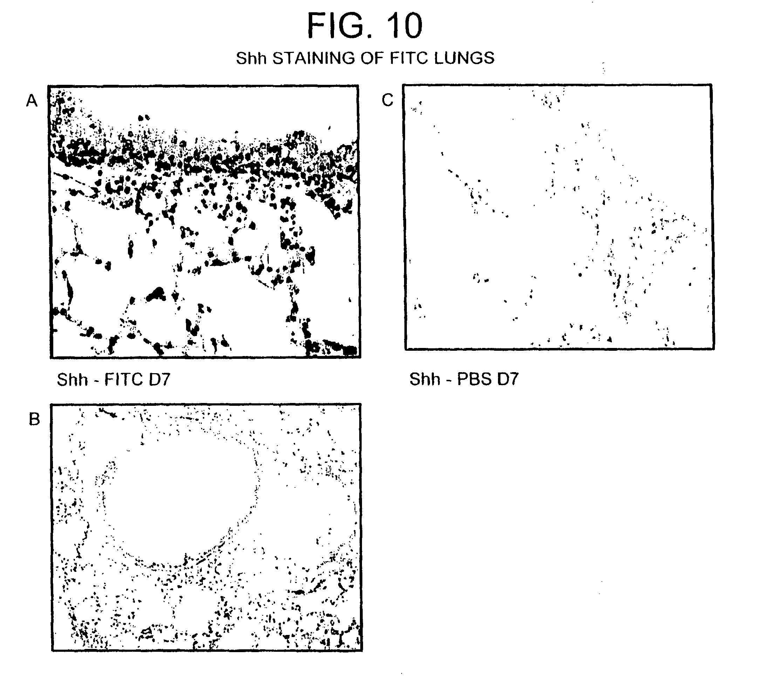

Mice were treated intratracheally with the hapten fluorescein isothiocyanate. Seven days later the lungs were removed and fixed in formalin. Sections were cut and stained for Shh by immunhistochemistry. Figs. 10A and 10B show expression of Shh in the lung of FITC treated mice, while Fig. 10C shows the staining for Shh observed in the control lung. Shh could be detected on epithelial cells, and a higher level of Shh was detected on a basal cell population in the lung interstitium consistent in morphology with fibroblasts.

### Example 5 : Epithelial cells express high levels of Shh following FITC damage

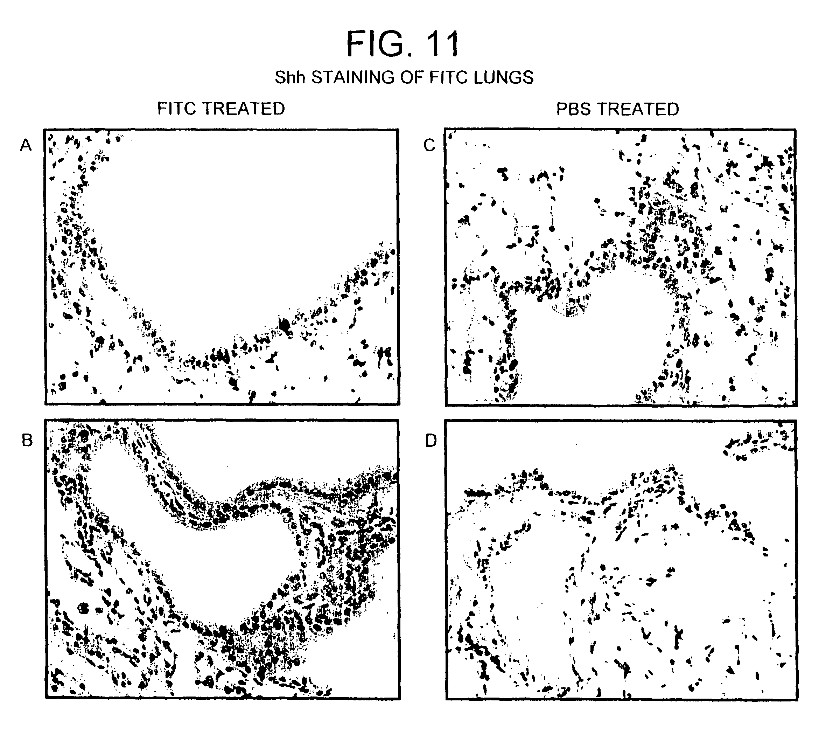

Mice were treated intratracheally with the hapten fluorescein isothiocyanate. One month later the lungs were removed and fixed in formalin. Sections were cut and stained for Shh by immunhistochemistry. Figs. 11A and 11B show expression of Shh in two different sections of lung of FITC treated mice, while Figs. 11C and 11D show the staining for Shh observed in the control lung. Shh could be detected at a higher level on epithelial cells than on a basal cell population in the lung interstitium.

### Example 6 : Epithelial cells express high levels of Ptc following FITC damage

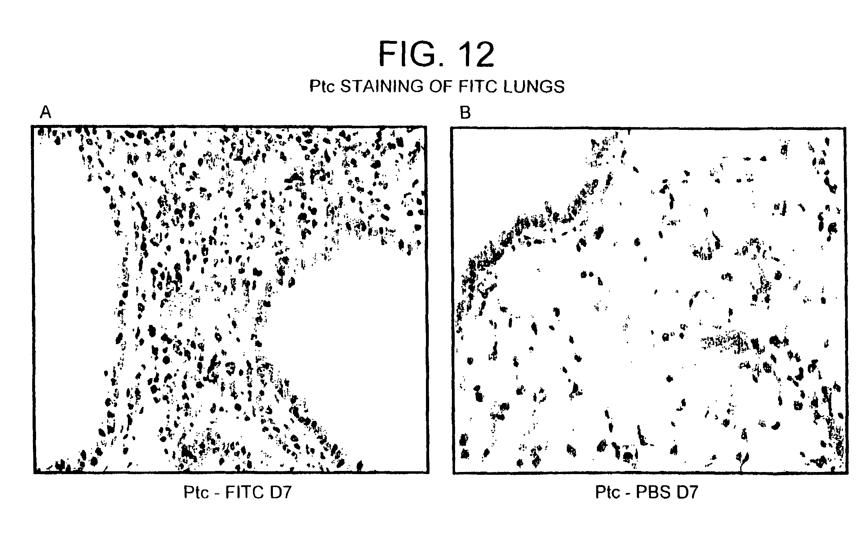

Mice were treated intratracheally with the hapten fluorescein isothiocyanate. Seven days later the lungs were removed and fixed in formalin. Sections were cut and stained for Shh by immunhistochemistry. Fig. 12A shows expression of Ptc in the lung of FITC treated mice, while Fig. 12B shows the staining for Ptc observed in the control lung. Ptc could be detected on epithelial cells and a higher level of Ptc was detected on infiltrating leukocytes found in the lung interstitium.

### Example 7 : Shh and Ptc staining on biopsy material from human CFA lung

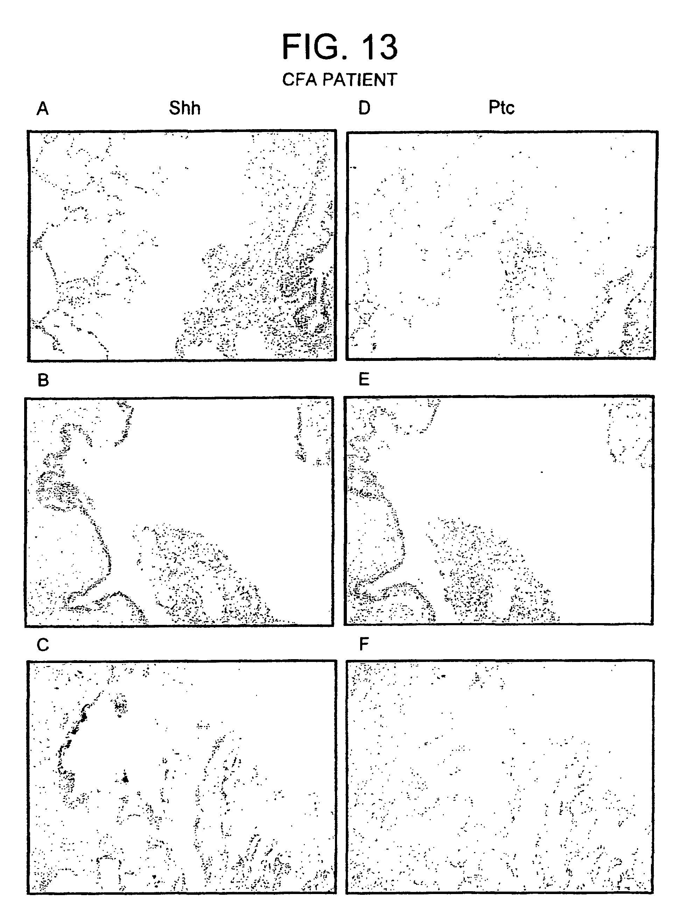

Archive material from a CFA patient was sectioned and stained by immunohistochemistry for the presence of Shh-N (the bioactive protein) and Ptc. Figs. 13A-C show staining for Shh and Figs. 13D-F show Ptc expression. Each Fig. represents a serial section taken from the same piece of lung at 10x. The cells within the airway interstitium and aveolar space contain leukocytes and these stain strongly for Ptc.

### Example 8 : Shh and Ptc staining on biopsy material from human CFA lung

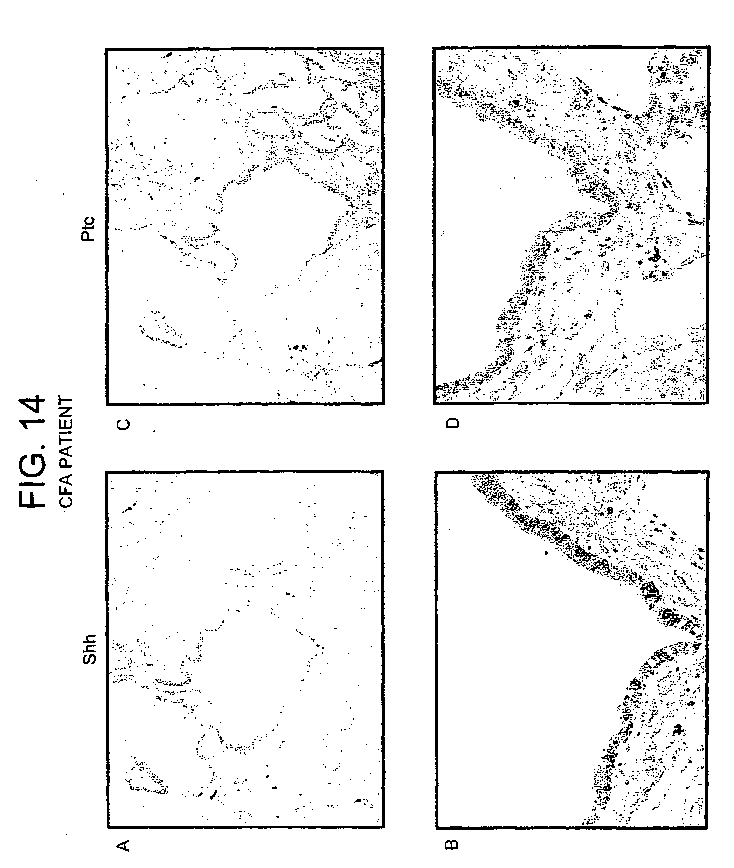

Archive material from a CFA patient was sectioned and stained by immunohistochemistry for the presence of Shh-N (the bioactive protein) and Ptc. Figs. 14A and B show staining for Shh and Figs. 14C and D show Ptc expression. Figs. 14A and C represents a serial section taken from the same piece of lung at 10x, while Figs. 14B and D are 40x views taken from the lower portion of the section. The cells within the airway interstitium and aveolar space contain leukocytes and these stain strongly for Ptc.

### Example 9 : Shh and Ptc staining on biopsy material from human CFA lung

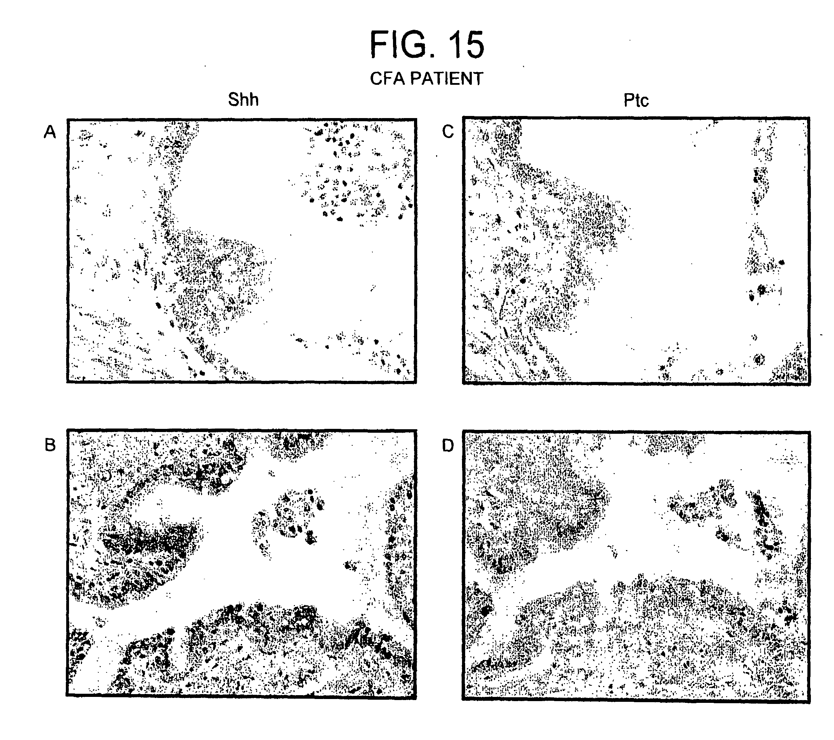

Archive material from a CFA patient was sectioned and stained by immunohistochemistry for the presence of Shh-N (the bioactive protein) and Ptc. Figs. 15A and B show staining for Shh and Figs. 15C and D show Ptc expression. Figs. 15A and C represent serial sections taken from the same piece of lung, while Figs. 15B and D are serial sections taken from another section. The cells within the airway lumen contain aveolar macrophages and these stain strongly for Ptc.

### Example 10 : Effect of introduction of SPC-HIP

Our previous studies have revealed that dysregulation of the Shh signalling pathway during epithelial cell repair in the lung, can lead to lymphocyte infiltration with concomitant induction of interstitial fibrosis and scarring. We have previously used a novel model of pulmonary fibrosis where naive BALB/c mice were treated i.t. with 50 µg of FITC dissloved in saline (PBS) leads to an initial strong inflammatory response which resolves by day 7, but EC hyperplasia is evident at ths time and lymphocytes begin to infiltrate the lung at the sites of EC hyperplasia by day 21. By day 28 there is evidence of interstitial fibrosis which seems to be aggravated by the presence of lymphocytes in the lung. In this model we have observed increased expression of Ptc-1 in sites of EC hyperplasia but not in normal areas of lung tissue. This indicates that there is dysregulation of the Shh pathway in the disease process in the FITC treated mice.

Since HIP is a natural antagonist of the Shh protein, we overexpress HIP in the lung using the SPC expresson vector as follows:

BALB/c mice are treated i.t. with 50 µg of FITC dissloved in saline (PBS) and 1-3 months later mice, which are time points where it is known that mice would normally have mild to severe fibrosis respectively, they are given two injections of either SPC plasmid alone in saline or SPC-HIP in saline i.t. 7 days apart. Mice are sacrificed at day 7, day 30 and day 60 post SPC-HIP administration. The lung tissue is removed and fixed in 4% buffered formalin. Sections are examined by H & E, PAS, Masons-trichrome and Ki-67 at the various time points. In addition, we examine the expression of Ptc and Shh by ISH and immunohistochemistry. A reduction of Ptc expression and EC hyperplasia and lung fibrosis is seen when compared to the epithelium of control mice.

### Example 11 : Effect of introduction of PKA

BALB/c mice are treated i.t. with 50 µg of FITC dissloved in saline (PBS) and 1-3 months later mice, which are time points where it is known that mice would normally have mild to severe fibrosis respectively, they are given two injections of PKA in saline i.t. 7 days apart. Mice are sacrificed at day 7, day 30 and day 60 post SPC-PKA administration. The lung tissue is removed and fixed in 4% buffered formalin. Sections are examined by H & E, PAS, Masons-trichrome and Ki-67 at the various time points. In addition, we examine the expression of Ptc and Shh by ISH and immunohistochemistry. A reduction of Ptc expression and EC hyperplasia and lung fibrosis is seen when compared to the epithelium of control mice.

### Example 12 : Epithelial cells express high level of Dvl-1 following FITC damage

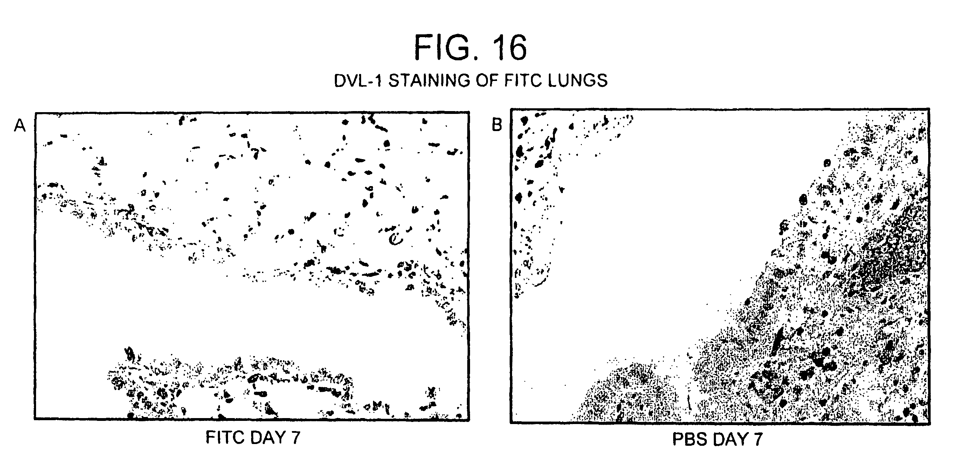

Mice were treated intratracheally with the hapten fluorescein isothiocyanate. Seven days later the lungs were removed and fixed in formalin. Sections were cut and stained for Dvl-1 by immunhistochemistry. Fig. 16A shows expression of Dvl-1 in the of lung of FITC treated mice, while Fig. 16B shows the staining for Dvl-1 observed in the control lung. Strong expression of Dvl-1 on epithelial cells and infiltrating leukocytes was observed.

### Example 13 : Dvl-1 adenovirus induces epithelial cell proliferation

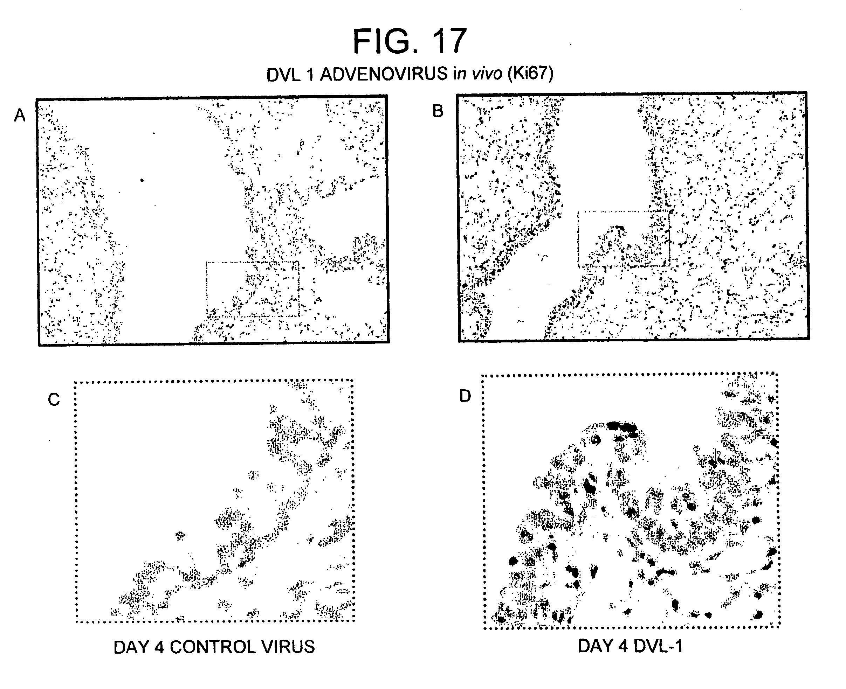

Mice were treated intratracheally with a control adenovirus (Figs. 17A and C) or an adnenovirus containing the murine Dvl-1 cDNA (Figs. 17B and D). Four days later the lungs were removed and fixed in formalin. Sections were cut and stained for the proliferation marker Ki67 by immunohistochemistry. Figs. 17A and B show a view of the lung at 10x and Figs. 17C and D are 40x views of the sections shown in the dotted box. Proliferating cells express the Ki67 antigen at high levels.

### Example 14 : A role for dysregulation of Wnt signalling in lung fibrosis

The Dvl-1 protein is overexpressed in the lung epithelia of mice to examine what effect it may have on the development of lung fibrosis.

BALB/c mice were injected i.t. with either (i) 20 µg of SPC plasmid dissolved in saline, or (ii) 20 µg of SPC-Dvl-1 plasmid DNA dissolved in saline on day 0 and day 7. Mice were sacificed at day 14 and day 35 where upon the lungs were removed and placed into 4% buffered formalin.

The lung tissue is removed and fixed in 4% buffered formalin. Sections are examined by H & E, PAS, Masons-trichrome and Ki-67 at the various time points. In addition, the expression of Dvl-1, Ptc and Shh is examined by ISH and immunohistochemistry. It was found that overexpression leads to EC hyperplasia and lung fibrosis.

### Example 15 : To examine the effect of introduction of the Wnt antagonist WIF-1

WIF-1 is a novel protein that was recently identified to be expressed as a transmembrane protein which binds to Wnt proteins to neuralize them. WIF-1 is normally expressed in the lung tissue. as well as the brain and so we performed a similar series of experiments as for the SPC-HIP protocols using SPC-WIF-1 in its place. Again a reduction in EC hyperplasia and lung fibrosis is seen when compared to the epithelium of control mice.

SEQ ID NOS: 1 & 2
DEFINITION   M.musculus (C57BL/6J) Shh mRNA.
ACCESSION    X76290
NID          g2597987
KEYWORDS     shh gene; sonic hedgehog protein.
SOURCE       house mouse.
  ORGANISM   <a href=/htbin-post/Taxonomy/wgetorg?id=10090>Mus musculus</a>
             Eukaryota; Metazoa; Chordata; Vertebrata; Mammalia; Eutheria;
             Rodentia; Sciurognathi; Muridae; Murinae; Mus.
FEATURES             Location/Qualifiers
     source          1..1314
                     /organism="Mus musculus"
/gene="Shh"
                     /codon_start=1
                     /product="sonic hedgehog"
/translation="MLLLLARCFLVILASSLLVCPGLACGPGRGFGKRRHPKKLTPLA
                     YKQFIPNVAEKTLGASGRYEGKITRNSERFKELTPNYNPDIIFKDEENTGADRLMTQR
                     CKDKLNALAISVMNQWPGVKLRVTEGWDEDGHHSEESLHYEGRAVDITTSDRDRSKYG
                     MLARLAVEAGFDWVYYESKAHIHCSVKAENSVAAKSGGCFPGSATVHLEQGGTKLVKD
                     LRPGDRVLAADDQGRLLYSDFLTFLDRDEGAKKVFYVIETLEPRERLLLTAAHLLFVA
                     PHNDSGPTPGPSALFASRVRPGQRVYVVAERGGDRRLLPAAVHSVTLREEEAGAYAPL
                     TAHGTILINRVLASCYAVIEEHSWAHRAFAPFRLAHALLAALAPARTDGGGGGSIPAA
                     QSATEARGAEPTAGIHWYSQLLYHIGTWLLDSETMHPLGMAVKSS"

                     /gene="Shh"
                     /product="sonic hedgebog"
BASE COUNT      256 a    411 c    427 g    220 t
ORIGIN
        1 atgctgctgc tgctggccag atgttttctg gtgatccttg cttcctcgct gctggtgtgc
       61 cccgggctgg cctgtgggcc cggcaggggg tttggaaaga ggcggcaccc caaaaagctg
      121 acccctttag cctacaagca gtttattccc aacgtagccg agaagaccct aggggccagc
      181 ggcagatatg aagggaagat cacaagaaac tccgaacgat ttaaggaact cacccccaat
      241 tacaaccccg acatcatatt taaggatgag gaaaacacgg gagcagaccg gctgatgact
      301 cagaggtgca aagacaagtt aaatgccttg gccatctctg tgatgaacca gtggcctgga
      361 gtgaagctgc gagtgaccga gggctgggat gaggacggcc atcattcaga ggagtctcta
      421 cactatgagg gtcgagcagt ggacatcacc acgtccgacc gggaccgcag caagtacggc
      481 atgctggctc gcctggctgt ggaagcaggt ttcgactggg tctactatga atccaaagct
      541 cacatccact gttctgtgaa agcagagaac tccgtggcgg ccaaatccgg cggctgtttc
      601 ccgggatccg ccaccgtgca cctggagcag ggcggcacca agctggtgaa ggacttacgt
      661 cccggagacc gcgtgctggc ggctgacgac cagggccggc tgctgtacag cgacttcctc
      721 accttcctgg accgcgacga aggcgccaag aaggtcttct acgtgatcga gacgctggag
      781 ccgcgcgagc gcctgctgct caccgccgcg cacctgctct tcgtggcgcc gcacaacgac
      841 tcggggccca cgcccgggcc aagcgcgctc tttgccagcc gcgtgcgccc cgggcagcgc
      901 gtgtacgtgg tggctgaacg cggcggggac cgccggctgc tgcccgccgc ggtgcacagc
      961 gtgacgctgc gagaggagga ggcgggcgcg tacgcgccgc tcacggcgca cggcaccatt
     1021 ctcatcaacc gggtgctcgc ctcgtgctac gctgtcatcg aggagcacag ctgggcacac
     1081 cgggccttcg cgcctttccg cctggcgcac gcgctgctgg ccgcgctggc acccgcccgc
     1141 acggacggcg gggcgggggg cagcatccct gcagcgcaat ctgcaacgga agcgaggggc
     1201 gcggagccga ctgcgggcat ccactggtac tcgcagctgc tctaccacat tggcacctgg
     1261 ctgttggaca gcgagaccat gcatcccttg ggaatggcgg tcaagtccag ctga

SEQ ID NOS: 3 & 4
DEFINITION  Mus musculus C57 Black/6 dishevelled segment polarity protein
            homolog (Dvl-1) mRNA, complete cds.
ACCESSION   U10115
NID         g497689
KEYWORDS    .
SOURCE      mouse.
  ORGANISM  <a href=/htbin-post/Taxonomy/wgetorg?id=10090>Mus musculus</a>
            Eukaryota; Metazoa; Chordata; Craniata; Vertebrata; Mammalia;
            Eutheria; Rodentia; Sciurognathi; Muridae; Murinae; Mus.
FEATURES             Location/Qualifiers
     source          1..3286
                     /organism="Mus musculus"
                     /strain="C57 Black/6"
/gene="Dvl-1"
/codon_start=1
/translation="MAETKIIYHMDEEETPYLVKLPVAPERVTLADFKNVLSNRPVHA
                     YKFFFKSMDQDFGVVKEEIFDDNAKLPCFNGRVVSWLVLAEGAHSDAGSQGTDSHTDL
                     PPPLERTGGIGDSRPPSFQPNVASSRDGMDNETGTESMVSHRRERARRRNRDEAARTN
                     GHPRGDRRRDLGLPPDSASTVLSSELESSSFIDSDEEDNTSRLSSSTEQSTSSRLVRK
                     HKCRRRKQRLRQTDRASSFSSITDSTMSLNIITVTLNMERHHFLGISIVGQSNDRGDG
                     GIYIGSIMKGGAVAADGRIEPGDMLLQVNDVNFENMSNDDAVRVLREIVSQTGPISLT
                     VAKCWDPTPRSYFTIPRADPVRPIDPAAWLSHTAALTGALPRYGTSPCSSAITRTSSS
                     SLTSSVPGAPQLEEAPLTVKSDMSAIVRVMQLPDSGLEIRDRMWLKITIANAVIGADV
                     VDWLYTHVEGFKERREARKYASSMLKHGFLRHTVNKITFSEQCYYVFGDLCSNLASLN
                     LNSGSSGASDQDTLAPLPHPSVPWPLGQGYPYQYPGPPPCFPPAYQDPGFSCGSGSAG
                     SQQSEGSKSSGSTRSSHRTPGREERRATGAGGSGSESDHTVPSGSGSTGWWERPVSQL
                     SRGSSPRSQASAVAPGLPPLHPLTKAYAVVGGPPGGPPVRELAAVPPELTGSRQSFQK
                     AMGNPCEFFVDIM"
     <a href=/cgi-bin/Entrez/getfeat?gi=497689&id=2&entity=1>polyA_site</a>        3286
                     /note="5 A residues"
BASE COUNT      679 a    969 c   1013 g    625 t
ORIGIN
        1 agcccgaggg gcggcgggcc gcgggagccc tcagagccgc tttccctggc gcccgctccg
       61 gggccgcggc ggatgggcgg ccgcgggccg cggggcgaca ggcggggaac gggtgcgagc
      121 cgggaccggg aggggcggcc gcgccaaggg gccgcggggc ggccgggcgg ggcgcgggcc
      181 ggcggtttgg gagggcgccc cgcgtccgag aggcgagccg ggccctgacg ccgcgcgggt
      241 tccgcgtcgc ccctgccgcg ccatggcgga gaccaaaatc atctaccaca tggacgagga
      301 ggagacgccg tacctggtca agctgcccgt agctcccgag cgcgtcacgc tggccgactt
      361 caagaacgtg ctcagcaacc ggccggtgca cgcctacaaa ttcttcttca agtccatgga
      421 ccaggacttc ggggtggtga aggaggagat cttcgatgac aatgccaagt tgccctgctt
      481 caatggccgg gtggtttcct ggctggtcct ggctgagggc gctcactcgg atgcagggtc
      541 ccagggcact gacagccaca cggacctgcc cccacccctt gagaggacag gcggcattgg
      601 ggactccagg cccccctcct tccaaccaaa tgttgccagt agccgggacg gaatggacaa
      661 tgagacaggc acagagtcta tggtcagtca ccggcgggag cgagcccgac gtcgaaaccg
      721 cgatgaggct gcccggacca atgggcaccc gagaggggat cggcggcggg acctgggact
      781 acctccagac agtgcatcta ctgtactgag cagtgagctt gaatctagca gctttattga
      841 ctcagatgag gaggacaata cgagccggct gagcagctcc acagagcaga gcacctcctc
      901 tcggctagtt cggaagcaca aatgccgtcg tcggaagcag cgcttgaggc agacagaccg
      961 ggcatcctcc ttcagcagca tcacagactc caccatgtcc ctgaacatca tcaccgtcac
     1021 tctcaacatg gagaggcacc acttcctggg catcagcatc gtgggccaga gcaacgaccg
     1081 gggtgatggc ggcatctaca ttggatccat catgaagggc ggggccgtgg ctgctgatgg
     1141 ccgcattgag ccgggcgaca tgttgctgca ggtgaacgat gtcaactttg agaacatgag
     1201 caatgacgac gctgtacggg tgcttcggga gatcgtgtcc cagacagggc ccatcagtct
     1261 cacagtggcc aagtgctggg acccaacccc tcggagctac ttcaccatcc caagggctga
     1321 cccagtgcga cccatcgacc cggctgcctg gctgtcccac acagcagcac tgacgggtgc
     1381 cctgccccgc tatggtacga gtccctgctc cagcgccatc acacgcacca gctcttcctc
     1441 actaaccagc tcagtgcctg gcgccccaca gcttgaggag gcgccgctga ctgtgaagag
     1501 tgacatgagt gccattgtcc gcgtcatgca gttgccagac tcaggactgg agatccggga
     1561 ccgcatgtgg cttaagatca ccattgccaa tgctgtcatt ggggcggatg tggtggactg
     1621 gctgtacaca cacgtggagg gcttcaagga gcgaagggag gcaagaaagt atgccagcag
     1681 tatgctgaag cacggtttcc tgaggcacac cgtgaacaag atcacctttt ctgagcagtg
     1741 ctactatgtc tttggcgacc tgtgcagtaa cctcgcatcc ctgaacctca acagtggctc
     1801 cagtggagcc tcagatcagg acacactggc cccactgccc cacccatcag taccctggcc
     1861 cttgggtcaa ggctacccct accagtaccc aggacccccg ccctgcttcc cacctgctta
     1921 ccaggaccct ggcttcagct gcggcagcgg cagtgctggg agccagcaga gtgaagggag

1981 caagagcagt gggtccacac ggagcagcca tcggacccca ggccgagagg agcgccgggc
2041 aactggagct gggggtagtg gcagtgaatc agaccacaca gtaccaagtg ggtctggtag
2101 caccggctgg tgggagcggc ctgtcagcca gcttagccgt ggcagtagcc ctcgaagtca
2161 ggcttcagct gttgccccag ggctcccccc actgcacccc cttacaaagg cctatgcagt
2221 agtgggtggg ccacctggag ggccacctgt ccgggagctg gctgctgtcc ctccagaact
2281 tacaggtagc cgccagtcct tccaaaaggc catgggaaac ccctgtgagt tctttgtgga
2341 catcatgtga tgatcaacca atgtcttcag cgctgcctgt ggctgagtct gagctcctgc
2401 tgtgccagga gctctgcgct ggccgtggtg gtggccagcc aggatagatc agctgtgggg
2461 tctgggccag ggcagaggga gcaggctcca gaggaggggc agagggcagc cataccacca
2521 ggatattggc ttgacatttt gtctgctctt ggggctgctg atggtggtac agctcaagta
2581 tctatagagt cttgtaagga gacatctctg actttaagtc ctcagcacaa gtctcaggga
2641 ccacctcctg gttcccttct ttggaagtga cctccattta gaacaagaaa ggctctcttg
2701 ggctcttggt accccttgcc ccaaagcctc acagaactgt gcacagggac acaggctgac
2761 tgtcgctaag ttcatgggcc tcacctgtca ggccaaggtg ggatttttga gggttagaca
2821 gaaccttcaa acctctctgg ctgcccaggt ggggtctaac ttatttattt attgctagcc
2881 tgcctgctct aagggtggca gctggttacc caaaggggca gtttgcatgc ccctttcccc
2941 acctgctact tggcacatga caacacagtt tgtactgaag gtatgtgaag ggtagctagt
3001 aggagagaca ggagagagac ctggcaccta gccactgtcc cagtctcagt ggtgggtgac
3061 agtgaacaca agagctgcag aggtgggacc ctgttctgtt tctgttctgg tggctgccca
3121 tcatcacgtg ccactgccat cccggcacag cggccccaca catctacact agacactgtg
3181 tcaaagtctg agtgactggg tagttgacat agagctgctt ctgtgtaaat gctgcttctg
3241 tgtaaatgct attttaaaca ctaaaaaagc gtttaatttt atggga

SEQ ID NOS: 5 & 6
DEFINITION  Mus musculus hedgehog-interacting protein (Hip) mRNA, complete cds.
ACCESSION   AF116865
NID         g4868121
KEYWORDS    .
SOURCE      house mouse.
  ORGANISM  <a href=/htbin-post/Taxonomy/wgetorg?id=10090>Mus musculus</a>
            Eukaryota; Metazoa; Chordata; Craniata; Vertebrata; Mammalia;
            Eutheria; Rodentia; Sciurognathi; Muridae; Murinae; Mus.
FEATURES             Location/Qualifiers
     source          1..2669
                     /organism="Mus musculus"
/gene="Hip"
                     /codon_start=1
                     /product="hedgehog-interacting protein"
/translation="MLKMLSFKLLLLAVALGFFEGDAKFGERSEGSGARRRRCLNGNP
                     PKRLKRRDRRVMSQLELLSGGEILCGGFYPRVSCCLQSDSPGLGRLENKIFSATNNSE
                     CSRLLEEIQCAPCSPHSQSLFYTPERDVLDGDLALPLLCKDYCKEFFYTCRGHIPGLL
                     QTTADEFCFYYARKDAGLCFPDFPRKQVRGPASNYLGQMEDYEKVGGISRKHKHNCLC
                     VQEVMSGLRQPVSAVHSGDGSHRLFILEKEGYVKILTPEGELFKEPYLDIHKLVQSGI
                     KGGDERGLLSLAFHPNYKKNGKLYVSYTTNQERWAIGPHDHILRVVEYTVSRKNPHQV
                     DVRTARVFLEVAELHRKHLGGQLLFGPDGFLYIILGDGMITLDDMEEMDGLSDFTGSV
                     LRLDVDTDMCNVPYSIPRSNPHFNSTNQPPEVFAHGLHDPGRCAVDRHPTDININLTI
                     LCSDSNGKNRSSARILQIIKGRDYESEPSLLEFKPFSNGPLVGGFVYRGCQSERLYGS
                     YVFGDRNGNFLTLQQSPVTKQWQEKPLCLGASSSCRGYFSGHILGFGEDELGEVYILS
                     SSKSMTQTHNGKLYKIVDPKRPLMPEECRVTVQPAQPLTSDCSRLCRNGYYTPTGKCC
                     CSPGWEGDFCRIAKCEPACRHGGVCVRPNKCLCKKGYLGPQCEQVDRNVRRVTRAGIL
                     DQIIDMTSYLLDLTSYIV"
BASE COUNT      660 a    722 c    672 g    615 t
ORIGIN
        1 gctgcagccg ccggcagagg agacctcagc atcctcggga gcccagcgcc gaccctgcct
       61 ccgcccggcc cgctgccgcc accgccgccc tttcggttcc tgctactgtc tcacctaaac
      121 aactcccatt cagcggacaa gcgaagttct atgactctct tctcctctcc ttcctcctct
      181 tcttccaact ccttctcctc ccacttccca accgctgtgg aaagccccta acccaacaga
      241 cgctggcaag gctgcggaca agtgtcaact gcactttatc ttgctgctcc tactgcccta
      301 aggcaaagtt gcatagctct acatctttct ttcccagcca cctccctctg cccccaagag
      361 cgtcccgccg ccccgcagca ctctcctgga gctgcgccct agtgcccctg ctgggcagtg
      421 gcctttcccc caccccatcc tcccgcgtcc tgcccttgct gctccgggca gacgatgctg
      481 aagatgctct cgtttaagct gc[?]actgctg gccgtggctc tgggcttctt tgaaggagat
      541 gcgaagtttg gggaaaggag cgaggggagc ggagcgagaa ggagacggtg cctgaatggg
      601 aaccccccaa agcgcctaaa gagaagggac aggcgggtga tgtcccagct ggagctgctc
      661 agtggaggag agatcctgtg tggtggcttc tacccacgag tatcttgctg cctgcagagt
      721 gacagccctg gattggggc[?] tctggagaac aagatctttt ctgccaccaa caactcagaa
      781 tgcagcaggc tgctggagga gatccaatgt gctccctgct ccccgcattc ccagagcctc
      841 ttctacacac ctgaaagaga tgtcctggat ggggacctag cacttccgct cctctgcaaa
      901 gactactgca aagaattctt ttatacttg[?] cgaggccata ttccaggtct tcttcaaaca
      961 actgctgatg aattttgctt ttactatgca agaaaagatg ctgggttatg ctttccagac
     1021 ttcccgagaa agcaagtcag aggaccagca tctaactact tgggccagat ggaagactac
     1081 gagaaagtgg gggggatcag cagaaaacac aaacacaact gcctctgtgt ccaggaggtc
     1141 atgagtgggc tgcggcagcc tgtgagcgct gtgcacagcg gggatggctc ccatcggctc
     1201 ttcattctag agaaggaagg ctacgtgaaa attctaaccc cagaaggaga actgttcaag
     1261 gagccttact tggacattca caaacttgtt caaagtggaa taaagggagg agacgaaagg
     1321 ggcctgctaa gcctggcatt ccatcccaat tacaagaaaa atggaaagct gtatgtgtct
     1381 tataccacca accaggaacg gtgggctatt gggcctcacg accacattct tcgggttgtg
     1441 gaatacacag tatccaggaa aaacccccat caagttgatg tgagaacagc cagggtgttt
     1501 ctggaagtcg cagagctcca ccgaaagcat cttgggggac agctgctctt tggtcctgat
     1561 ggctttttgt acatcatcct tggggatggt atgatcacat tggatgacat ggaagagatg
     1621 gatgggttaa gtgacttcac aggctctgtg ctgaggctgg acgtggacac cgacatgtgc
     1681 aatgtgcctt attccatacc tcggagtaac cctcacttca acagcaccaa ccagccccca
     1741 gaagtatttg cccacggcct ccatgatcca ggcagatgtg ccgtggatcg acatcctact
     1801 gatataaaca tcaatttaac aatactttgc tcagattcca acgggaaaaa caggtcatca
     1861 gccagaatcc tacagataat aaagggaaga gattatgaaa gtgagccatc tcttcttgaa
     1921 ttcaagccat tcagtaacgg ccctttggtt ggtggatttg tttacagagg ctgtcagtct
     1981 gaaagattgt acggaagcta tgtgttcgga gatcgcaatg ggaatttctt aaccctccag
     2041 caaagcccag tgaccaagca atggcaagaa aagccgctct gcctgggtgc cagcagctcc
     2101 tgtcgaggct acttttcggg tcacatcttg ggatttggag aagatgaatt aggagaggtt

2161 tacattctat caagcagtaa gagtatgacc cagactcaca atggaaaact ctacaagatc
2221 gtagacccca aaagaccttt aatgcctgag gaatgcagag tcacagttca acctgcccag
2281 ccactgacct ccgattgctc ccggctctgt cgaaacggct actacacccc cactggcaag
2341 tgctgctgca gtcccggctg ggagggagac ttctgcagaa ttgccaagtg tgagccagcg
2401 tgccgtcatg gaggtgtctg tgtcagaccg aacaagtgcc tctgtaaaaa gggctatctt
2461 ggtcctcaat gtgaacaagt ggacaggaac gtccgcagag tgaccagggc aggtatcctt
2521 gatcagatca ttgacatgac gtcttacttg ctggatctca caagttacat tgtatagttt
2581 ctgggacagt tcgagtctat ctttccagtg ggcatttatt ttgaccttgt catcattaaa
2641 aagagagact gtccttctgc tacaaaaaa

SEQ ID NOS: 7 & 8
DEFINITION  Mus musculus Wnt inhibitory factor-1 mRNA, complete cds.
ACCESSION   AF122923
NID         g4585371
KEYWORDS    .
SOURCE      house mouse.
  ORGANISM  <a href=/htbin-post/Taxonomy/wgetorg?id=10090>Mus musculus</a>
            Eukaryota; Metazoa; Chordata; Craniata; Vertebrata; Mammalia;
            Eutheria; Rodentia; Sciurognathi; Muridae; Murinae; Mus.
FEATURES             Location/Qualifiers
     source          1..2047
                     /organism="Mus musculus"
/note="WIF-1; secreted protein; binds Wnt proteins and
                     inhibits their activities"
                     /codon_start=1
                     /product="Wnt inhibitory factor-1"
/translation="MARRRAFPAFALRLWSILPCLLLLPADAGQPPEESLYLWIDAHQ
                     ARVLIGFEEDILIVSEGKMAPFTHDFRKAQQRMPAIPVNIHSMNFTWQAAGQAEYFYE
                     FLSLRSLDKGIMADPTVNVPLLGTVPHKASVVQVGFPCLGKQDGVAAFEVNVIVMNSE
                     GNTILRTPQNAIFFKTCQQAECPGGCRNGGFCNERRVCECPDGFYGPHCEKALCIPRC
                     MNGGLCVTPGFCICPPGFYGVNCDKANCSTTCFNGGTCFYPGKCICPPGLEGEQCELS
                     KCPQPCRNGGKCIGKSKCKCPKGYQGDLCSKPVCEPGCGAHGTCHEPNKCQCREGWHG
                     RHCNKRYGASLMHAPRPAGAGLERHTPSLKKAEDRRDPPESNYIW"
BASE COUNT      567 a    476 c    486 g    518 t
ORIGIN
        1 agtaggaaca gcttcagccc cctcacctgc agccaaggcg agaacttcac aagcagcaca
       61 ggttgggtcc ctgctgcagg acttgcacca ccagcgacaa ggtcctgagc accatggctc
      121 ggagaagagc cttccctgct ttcgcgctcc ggctctggag catcctacct tgcctgctcc
      181 tgctgcgacc ggatgcaggg cagcccacctg aggagagctt gtacctgtgg atcgacgccc
      241 atcaggctag agtgctcata ggatttgaag aagacattct gattgtctcg gaggggaaaa
      301 tggccccctt tacacatgat ttcaggaaag cccaacaaag aatgccagcc attcctgtca
      361 atatccactc catgaatttt acctggcaag ctgcggggca ggcagaatac ttctacgagt
      421 tcctgtctct gcgctccctg gataaaggca tcatggcaga tccaactgtc aatgtccctt
      481 tgctgggaac agtgcctcac aaggcatcag ttgttcaagt tggtttcccg tgtctcggca
      541 aacaagacgg ggtagcagca tttgaagtga atgtgattgt catgaattct gaaggcaaca
      601 ccatccttag gacccctcag aatgccatct tctttaaaac atgtcaacaa gctgagtgtc
      661 ccggagggtg tcgaaatgga ggcttttgta acgaaaggcg ggtctgcgag tgtccggatg
      721 ggttctacgg gcctcactgt gagaaagccc tgtgcatacc ccgatgtatg aacggtggtc
      781 tgtgtgtcac tcctggcttc tgcatctgcc cccctggatt ctacggtgtc aactgtgaca
      841 aagcaaactg ctcaaccacc tgctttaatg gagggacctg cttttacccg ggaaaatgta
      901 tttgccctcc tggactcgag ggagagcagt gtgaactcac caaatgcccc caaccctgcc
      961 gaaatggagg taaatgcatt ggtaaaagca agtgtaagtg ccccaaaggt taccaaggag
     1021 acctgtgctc taagcccgtc tgcgagcctg gctgcggtgc ccacggaacc tgccacgaac
     1081 ccaacaagtg ccagtgtcga gagggctggc acggcagaca ctgcaataag aggtatggag
     1141 ccagcctcat gcatgccccg aggccagcag gcgccgggct ggagcgacac acgccttcac
     1201 ttaaaaaggc tgaggataga agggatccac ctgaatccaa ttacatctgg tgaaccccta
     1261 ccccaccatc tgaaacggtt caagttacac cgggttcaca gcctttgtta acctttcgcg
     1321 tgttggatgt tcaaatgctg ttcattacac tttagaaccc cggcctgaat tttattagct
     1381 tcattataaa tcactgggct catatctact cttcctttta ggttttctaa gcgtgtctag
     1441 catgatggta tagatttttt tccttcagtc cttttgggac agatcttata ttgtgtcagt
     1501 tgatcaggtt aaaaagaaaa aaaaatatct gtcttttcag tgtgtagttg acagatactt
     1561 gcaaaatcac aacacatttg tggtcttaga atcgggactg ttagagaggt taaactgggc
     1621 agagatgcat aaattacaag gtttcggata aagccaatag cagcgtttaa gctacagtat
     1681 ttccaatttt attgtcaaat atttggacat ctgtctaatt aatacttcaa ttgccccccc
     1741 ccccatcttg aatgcataca atctatttca cccttgctgt tactctagac agttcagttt
     1801 tgatggggcg ggggacaaag tttaaaaaaa ttacactgag ttagcggcat ttaaacaata
     1861 taatatattg taaacacgac gagataagga atataatgta tgaagccttt gcattggatg
     1921 gaagcaatat aatatattgt aaataaaaca cagctcttac atagtaaacg ttttatactg
     1981 tttgtatgta tgaaataaag gtgacgcttt cactttcaaa aaaaaaaaaa aaaaaaaaaa
     2041 aaaaaaa

### References

Ingham (1995) Curr Opin Genet and Dev 5:492-498

Chuang and McMahon (1999) Nature 397:617-621

Pepicelli *et al* (1998) Curr Biol 8(19):1083-1086

Hammerschmidt *et al* (1997) Trends Genet 13(1):14-21

Valenzuela *et al* (1995) J. Neurosci 15:6077-6084

Sasai *er al* (1994) Cell 79:779-790

Iemura *et al* (1998) PNAS 95:9337-9342

Kohler and Milstein (1975) Nature 256: 495-497

Davis *et al* Basic Methods in Molecular Biology (1986)

Sambrook *et al* Molecular Cloning: A Laboratory Manual, 2nd Edn, Cold Spring Harbor Laboratory Press, Cold Spring Harbor, NY (1989)

Sussman *et al* (1994) Dev. Bio. 166:73-86

Leyns *et al* Cell (1997) 88(6):747-56

Bouwmeester *et al* Nature (1996) 382(6592):596-601

Hsu *et al* Mol Cell (1998) 1(5):673-83

Hsieh *et al* Nature (1999) 398(6726):431-6

Development (1997) 124:53-63

Korfhagen *et al* (1990) PNAS 87:6122-61226

Cooper *et al* (1998) Science 280(5369):1603-7

Coventry *et al* (1998) Pediatr. Dev. Pathol. 1:29-41

## Claims

1. Use of an inhibitor of a Hedgehog signalling pathway in the preparation of a medicament for treatment of epithelial cell hyperplasia, fibrosis of tissue, inflammation or an immune disorder.

2. Use according to claim 1 in which the immune disorder is an autoimmune disease or graft rejection.

3. Use according to claim 2 in which the autoimmune disease is thyroiditis, insultitis, multiple sclerosis, iridocyclitis, uveitis, orchitis, hepatitis, Addison's disease, myasthenia gravis, rheumatoid arthritis or lupus erythematosus.

4. Use of any one of the preceding claims wherein the Hedgehog signalling pathway is the Sonic hedgehog, Indian hedgehog or Desert hedgehog signalling pathway.

5. Use of any one of the preceding claims in which the inhibitor is HIP, Fzb, Cerberus, WIF-1, Xnr-3, Noggin, Chordin, Gremlin, or Follistatin or a derivative, fragment, variant, mimetic, homologue or analogue thereof.

6. Use of any one of claims 1 to 4 in which the inhibitor is Ptc, Cos2 or PKA.

7. Use of any one of claims 1 to 4 wherein the inhibitor is an antibody.

8. Use of any preceding claim for the preparation of a medicament for the treatment of the lung or kidney.

9. Use of any preceding claim for the preparation of a medicament for the treatment of adult respiratory distress syndrome; chronic obstructive airway disorders including asthma, emphysema and chronic bronchitis; atelectasis; occupational lung disease including silicosis; hypersensitivity diseases of the lung including hypersensitivity pneomonitis; idiopathic interstitial lung diseases including idiopathic pulmonary fibrosis, pneumonia including usual interstitial pneumonia, desquamative interstitial pneumonia and acute interstitial pneumonia; and pleural fibrosis.

10. Use as claimed in any one of the preceding claims wherein the inhibitor is an antagonist selected from polypeptides and fragments thereof, linear peptides, cyclic peptides, synthetic and natural compounds including low molecular weight organic or inorganic compounds.

## Patentansprüche

1. Verwendung eines Inhibitors für einen Hedgehog-Signalweg bei der Herstellung eines Medikaments für die Behandlung von Epithelzellhyperplasie, Gewebefibrose, Entzündung oder einer Immunstörung.

2. Verwendung nach Anspruch 1, wobei die Immunstörung eine Autoimmunerkrankung oder Transplantatabstoßung ist.

3. Verwendung nach Anspruch 2, wobei die Autoimmunerkrankung Thyroiditis, Insulitis, Multiple Sklerose, Iridocyclitis, Uveitis, Orchitis, Hepatitis, Addison-Krankheit, Myasthenia gravis, rheumatoide Arthritis oder Lupus Erythematosus ist.

4. Verwendung nach einem der vorangegangenen Ansprüche, wobei der Hedgehog-Signalweg der Sonic-Hedgehog-, Indian-Hedgehog- oder Desert-Hedgehog-Signalweg ist.

5. Verwendung nach einem der vorangegangenen Ansprüche, wobei der Inhibitor HIP, Fzb, Cerberus, WIF-1, Xnr-3, Noggin, Chordin, Gremlin oder Follistatin oder ein Derivat, ein Fragment, eine Variante, ein Nachahmer, ein Homologes oder ein Analoges davon ist.

6. Verwendung nach einem der Ansprüche 1 bis 4, wobei der Inhibitor Ptc, Cos2 oder PKA ist.

7. Verwendung nach einem der Ansprüche 1 bis 4, wobei der Inhibitor ein Antikörper ist.

8. Verwendung nach einem der vorangegangenen Ansprüche für die Herstellung eines Medikaments zur Behandlung der Lunge oder der Niere.

9. Verwendung nach einem der vorangegangenen Ansprüche für die Herstellung eines Medikaments zur Behandlung von Atemnotsyndrom beim Erwachsenen, chronischer, hinderlicher Atemwegsstörungen, einschließlich Asthma, Emphysem und chronische Bronchitis, Atelektase, berufsbedingte Lungenerkrankung, einschließlich Silikose, Überempfindlichkeitserkrankungen der Lunge, einschließlich Überempfindlichkeitslungenentzündung; idiopathische interstitielle Lungenerkrankungen, einschließlich idiopathischer pulmonaler Fibrose, Lungenentzündung, einschließlich herkömmlicher interstitieller Lungenentzündung, desquamativer interstitieller Lungenentzündung und akuter interstitieller Lungenentzündung, und pleurale Fibrose.

10. Verwendung nach einem der vorangegangenen Ansprüche, wobei der Inhibitor ein Antagonist ist, ausgewählt unter Polypeptiden und Fragmenten davon, linearen Peptiden, zyklischen Peptiden, synthetischen und natürlichen Verbindungen, einschließlich niedermolekularen organischen oder anorganischen Verbindungen.

## Revendications

1. Utilisation d'un inhibiteur d'une voie de signalisation Hedgehog dans la préparation d'un médicament destiné au traitement d'hyperplasie des cellules épithéliales, de fibrose tissulaire, d'inflammation ou d'un trouble immunitaire.

2. Utilisation selon la revendication 1, dans laquelle le trouble immunitaire est une maladie auto-immune ou un rejet de greffe.

3. Utilisation selon la revendication 2, dans laquelle la maladie auto-immune est la thyroïdite, l'insulite, la sclérose en plaques, l'iridocyclite, l'uvéite, l'orchite, l'hépatite, la maladie d'Addison, la myasthénie grave, la polyarthrite rhumatoïde ou le lupus érythémateux.

4. Utilisation selon l'une quelconque des revendications précédentes, dans laquelle la voie de signalisation Hedgehog est la voie de signalisation Sonic Hedgehog, Indian Hedgehog ou Desert Hedgehog.

5. Utilisation selon l'une quelconque des revendications précédentes, dans laquelle l'inhibiteur est HIP, Fzb, Cerberus, WIF-1, Xnr-3, Noggine, Chordine, Gremlin ou Follistatine ou un dérivé, fragment, variant, mimétique, homologue ou analogue de ceux-ci.

6. Utilisation selon l'une quelconque des revendications 1 à 4, dans laquelle l'inhibiteur est Ptc, Cos2 ou PKA.

7. Utilisation selon l'une quelconque des revendications 1 à 4, dans laquelle l'inhibiteur est un anticorps.

8. Utilisation selon l'une quelconque des revendications précédentes pour la préparation d'un médicament destiné au traitement du poumon ou du rein.

9. Utilisation selon l'une quelconque des revendications précédentes, pour la préparation d'un médicament destiné au traitement du syndrome de détresse respiratoire de l'adulte ; de troubles chroniques obstructifs des voies aériennes comprenant l'asthme, l'emphysème et la bronchite chronique ; de l'atélectasie ; d'une maladie pulmonaire professionnelle comprenant la silicose ; des maladies d'hypersensibilité du poumon comprenant la pneumopathie par hypersensibilité ; des pneumopathies interstitielles idiopathiques comprenant la fibrose pulmonaire idiopathique, une pneumonie comprenant la pneumonie interstitielle commune, la pneumonie interstitielle desquamative et la pneumonie interstitielle aiguë ; et la fibrose pleurale.

10. Utilisation selon l'une quelconque des revendications précédentes, dans laquelle l'inhibiteur est un antagoniste choisi parmi des polypeptides et leurs fragments, des peptides linéaires, des peptides cycliques, des composés synthétiques et naturels comprenant des composés inorganiques ou organiques de bas poids moléculaire.
